(19) Europäisches Patentamt
European Patent Office
Office européen des brevets

(11) EP 1 757 587 A1

(12) EUROPEAN PATENT APPLICATION

(43) Date of publication:
**28.02.2007 Bulletin 2007/09**

(21) Application number: **05384005.4**

(22) Date of filing: **15.07.2005**

(51) Int Cl.:
***C07D 231/06*** *(2006.01)* ***A61K 31/4155*** *(2006.01)*
***A61P 25/00*** *(2006.01)*

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(71) Applicant: **LABORATORIOS DEL DR. ESTEVE, S.A.**
**08041 Barcelona (ES)**

(72) Inventor: **Cuberes Altisen, Maria Rosa**
**San Cugat del Valles (Barcelona) (ES)**

# (54) Substituted pyrazoline compounds, their preparation and use as medicaments

(57) The present invention relates to substituted pyrazoline compounds, methods for their preparation, medicaments comprising these compounds as well as their use for the preparation of a medicament for the treatment of humans and animals.

**Figure 1:**

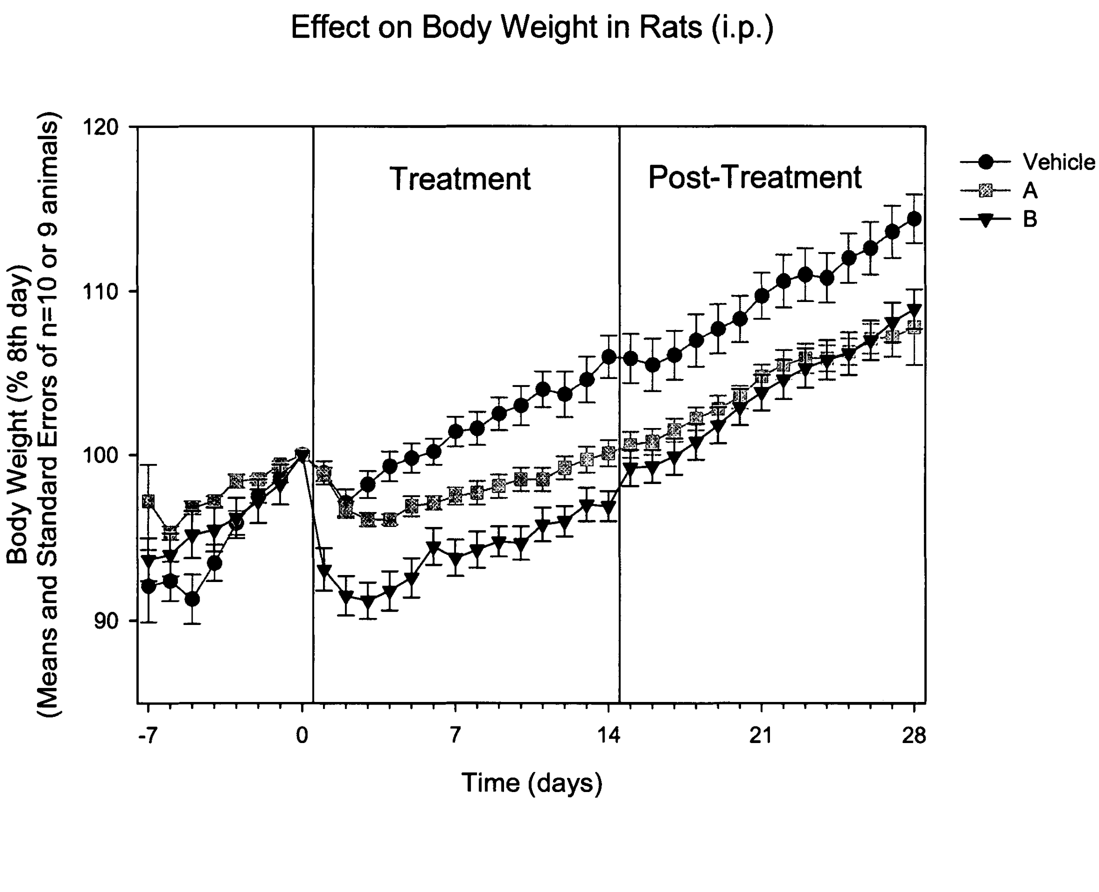


EP 1 757 587 A1

## Description

The present invention relates to substituted pyrazoline compounds, methods for their preparation, medicaments comprising these compounds as well as their use for the preparation of a medicament for the treatment of humans and animals.

Cannabinoids are compounds, which are derived from the cannabis sativa plant which is commonly known as marijuana. The most active chemical compound of the naturally occurring cannabinoids is tetrahydrocannabinol (THC), particularly $\Delta^9$-THC.

These naturally occuring cannabinoids as well as their synthetic analogues promote their physiological effects via binding to specific G-coupled receptors, the so-called cannabinoid-receptors.

At present, two distinct types of receptors that bind both the naturally occurring and synthetic cannabinoids have been identified and cloned. These receptors, which are designated $CB_1$ and $CB_2$ are involved in a variety of physiological or pathophysiological processes in humans and animals, e.g. processes related to the central nervous system, immune system, cardiovascular system, endocrinous system, respiratory system, the gastrointestinal tract or to reproduction, as described for example, in Hollister, Pharm. Rev. 38, 1986, 1-20; Reny and Singha, Prog. Drug. Res., 36, 71-114, 1991; Consroe and Sandyk, in Marijuana/Cannabinoids, Neurobiology and Neurophysiology, 459, Murphy L. and Barthe A. Eds., CRC Press, 1992.

Therefore, compounds, which have a high binding affinity for these cannabinoid receptors and which are suitable for modulating these receptors are useful in the prevention and/or treatment of cannabinoid-receptor related disorders.

In particular, the $CB_1$-Receptor is involved in many different food-intake related disorders such as bulimia or obesity, including obesity associated with type II diabetes (non-insulin-dependent diabetes) and thus, compounds suitable for regulating this receptor may be used in the prophylaxis and/or treatment of these disorders.

Thus, it was an object of the present invention to provide novel compounds for use as active substances in medicaments. In particular, these active substances should be suitable for the modulation of Cannabinoid receptors, more particularly for the modulation of Cannabinoid 1 ($CB_1$) receptors.

Said object was achieved by providing the substituted pyrazoline compounds of general formula I given below, their stereoisomers, corresponding salts and corresponding solvates thereof.

It has been found that these compounds have a high affinity for cannabinoid receptors, particularly for the $CB_1$-receptor, and that they act as modulators e.g. antagonists, inverse agonists or agonists on these receptors. They are therefore suitable for the prophylaxis and/or treatment of various disorders related to the central nervous system, the immune system, the cardiovascular system, the endocrinous system, the respiratory system, the gastrointestinal tract or reproduction in humans and/or animals, preferably humans including infants, children and grown-ups.

Thus, in one of its aspects the present invention relates to substituted pyrazoline compounds of general formula I,

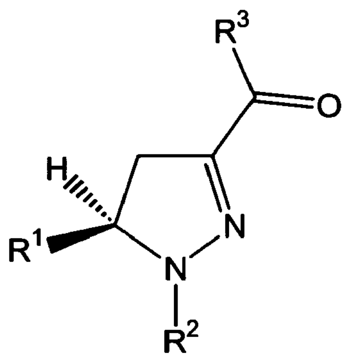

I

wherein

$R^1$ represents an optionally at least mono-substituted phenyl group,

$R^2$ represents an optionally at least mono-substituted phenyl group,

$R^3$ represents a saturated or unsaturated, optionally at least mono-substituted, optionally at least one heteroatom as ring member containing cycloaliphatic group, which may be condensed with an optionally at least mono-substituted

mono- or polycyclic ring system, or $R^3$ represents an optionally at least mono-substituted aryl or heteroaryl group, which may be condensed with an optionally at least mono-substituted mono- or polycyclic ring system, or $R^3$ represents an $-NR^4R^5$-moiety,

$R^4$ and $R^5$, identical or different, represent a hydrogen atom, an unbranched or branched, saturated or unsaturated, optionally at least mono-substituted aliphatic radical, a saturated or unsaturated, optionally at least mono-substituted, optionally at least one heteroatom as ring member containing cycloaliphatic group, which may be condensed with an optionally at least mono-substituted mono- or polycyclic ring system, or an optionally at least mono-substituted aryl or heteroaryl group, which may be condensed with an optionally at least mono-substituted mono- or polycyclic ring system and/or bonded via a linear or branched alkylene group, an $-SO_2-R^6$-moiety, or an $-NR^7R^8$-moiety,

$R^6$ represents a linear or branched, saturated or unsaturated, optionally at least mono-substituted aliphatic group, a saturated or unsaturated, optionally at least mono-substituted, optionally at least one heteroatom as ring member containing cycloaliphatic group, which may be condensed with a mono- or polycyclic ring-system, or an optionally at least mono-substituted aryl or heteroaryl group, which may be condensed with a mono- or polycyclic ring system and/or bonded via a linear or branched alkylene group,

$R^7$ and $R^8$, identical or different, represent a hydrogen atom, an unbranched or branched, saturated or unsaturated, optionally at least mono-substituted aliphatic radical, a saturated or unsaturated, optionally at least mono-substituted, optionally at least one heteroatom as ring member containing cycloaliphatic group, which may be condensed with an optionally at least mono-substituted mono- or polycyclic ring system, or an optionally at least mono-substituted aryl or heteroaryl group, which may be condensed with an optionally at least mono-substituted mono- or polycyclic ring system and/or bonded via a linear or branched alkylene group,

optionally in form of a corresponding N-oxide thereof, or a corresponding salt thereof, or a corresponding solvate thereof.

The following provisos (disclaimers) may apply for the pyrazoline compounds of general formula I given above, namely

that $R^4$ and $R^5$ do not both represent a hydrogen atom, and/or

that if one of the residues $R^4$ and $R^5$ represents a hydrogen atom or an alkyl group, which is optionally at least mono-substituted with an alkoxy group, an alkoxyalkoxy group, a halogen atom or a phenyl group, the other one of these residues $R^4$ and $R^5$ does not represent a pyrid-2-yl group, which is optionally mono-substituted in the 5-position, a pyrid-5-yl group, which is optionally mono-substituted in the 2-position, a pyrimid-5-yl group, which is optionally mono-substituted in the 2-position, a pyridaz-3-yl group, which is optionally mono-substituted in the 6-position, a pyrazin-5-yl group, which is optionally mono-substituted in the 2-position, a thien-2-yl group, which is optionally mono-substituted in the 5 position, a thien-2-yl group, which is optionally at least mono-substituted in the 4-position, a benzyl group, which is optionally mono-substituted in the 4-position of the ring, a phenethyl group, which is optionally mono-substituted in the 4-position of the ring, an optionally mono-, di- or tri-substituted phenyl group, a di-substituted phenyl group, wherein the two substituents together form an $-OCH_2O-$, $-OCH_2CH_2O-$ or $-CH_2CH_2O-$ chain, which is optionally substituted with one or more halogen atoms or one or two methyl groups, an -NH-phenyl-moiety, wherein the phenyl group may be mono-substituted in the 4-position, and/or

that if one of the residues $R^4$ and $R^5$ represents an alkynyl group, the other one of these residues $R^4$ and $R^5$ does not represent a phenyl group, which is optionally substituted in the 4-position, and/or

that if one of the residues $R^4$ and $R^5$ represents a hydrogen atom or a linear or branched, saturated or unsaturated, unsubstituted or substituted aliphatic radical, the other one of these residues $R^4$ and $R^5$ does not represent an unsubstituted or substituted thiazole group or an unsubstituted or substituted [1,3,4]thiadiazole group.

A mono- or polycyclic ring-system according to the present invention means a mono- or polycyclic hydrocarbon ring-system that may be saturated, unsaturated or aromatic. If the ring system is polycyclic, each of its different rings may show a different degree of saturation, i.e. it may be saturated, unsaturated or aromatic. Optionally each of the rings of the mono- or polycyclic ring system may contain one or more, e.g. 1, 2 or 3, heteroatoms as ring members, which may be identical or different and which can preferably be selected from the group consisting of N, O, S and P, more preferably be selected from the group consisting of N, O and S. Preferably the polycyclic ring-system may comprise two rings that are condensed. The rings of the mono- or polycyclic ring-sytem are preferably 5- or 6-membered.

The term "condensed" according to the present invention means that a ring or ring-system is attached to another ring or ring-system, whereby the terms "annulated" or "annelated" are also used by those skilled in the art to designate this kind of attachment.

If one or more of the residues $R^3$—$R^8$ represents or comprises a saturated or unsaturated, optionally at least one heteroatom as ring member containing cycloaliphatic group, which is substituted by one or more, e.g. 1, 2, 3 or 4, substituents, unless defined otherwise, each of the substituents may be independently selected from the group consisting

of hydroxy, fluorine, chlorine, bromine, branched or unbranched $C_{1-6}$-alkoxy, branched or unbranched $C_{1-6}$-alkyl, branched or unbranched $C_{1-4}$-perfluoroalkoxy, branched or unbranched $C_{1-4}$-perfluoroalkyl, oxo, amino, carboxy, amido, cyano, nitro, $-SO_2NH_2$, -CO-$C_{1-4}$-alkyl, -SO-$C_{1-4}$-alkyl, $-SO_2$-$C_{1-4}$-alkyl, -NH-$SO_2$-$C_{1-4}$-alkyl, wherein the $C_{1-4}$-alkyl may in each case be branched or unbranched, and a phenyl group, more preferably be selected from the group consisting of hydroxy, F, Cl, Br, methyl, ethyl, methoxy, ethoxy, oxo, $CF_3$ and a phenyl group.

If one or more of the residues $R^3$-$R^8$ represents or comprises a cycloaliphatic group, which contains one or more heteroatoms as ring members, unless defined otherwise, each of these heteroatoms may preferably be selected from the group consisting of of N, O and S. Preferably a cycloaliphatic group may contain 1, 2 or 3 heteratoms independently selected from the group consisting of N, O and S as ring members.

Suitable saturated or unsaturated, optionally at least one heteroatom as ring member containing, optionally at least mono-substituted cycloaliphatic groups may preferably be selected from the group consisting of Cyclopropyl, Cyclobutyl, Cyclopentyl, Cyclohexyl, Cycloheptyl, Cyclooctyl, Cyclopentenyl, Cyclohexenyl, Cycloheptenyl, Cyclooctenyl, Pyrrolidinyl, Piperidinyl, Piperazinyl, homo-Piperazinyl and Morpholinyl.

If one or more of the residues $R^3$-$R^8$ comprises a mono- or polycyclic ring system, which is substituted by one or more, e.g. 1, 2, 3, 4 or 5 substituents, unless defined otherwise, each of the substituents may be independently selected from the group consisting of hydroxy, fluorine, chlorine, bromine, branched or unbranched $C_{1-6}$-alkoxy, branched or unbranched $C_{1-6}$-alkyl, branched or unbranched $C_{1-4}$-perfluoroalkoxy, branched or unbranched $C_{1-4}$-perfluoroalkyl, amino, carboxy, oxo, amido, cyano, nitro, $-SO_2NH_2$, -CO-$C_{1-4}$-alkyl, -SO-$C_{1-4}$-alkyl, $-SO_2$-$C_{1-4}$-alkyl, -NH-$SO_2$-$C_{1-4}$-alkyl, wherein the $C_{1-4}$-alkyl may in each case be branched or unbranched, and a phenyl group, more preferably be selected from the group consisting of hydroxy, F, Cl, Br, methyl, ethyl, methoxy, ethoxy, $CF_3$, oxo and a phenyl group.

If one or more of the residues $R^1$-$R^8$ represents or comprises an aryl group, including a phenyl group, which is substituted by one or more, e.g. 1, 2, 3, 4 or 5 substituents, unless defined otherwise, each of the substituents may be independently selected from the group consisting of a halogen atom (e.g. F, Cl, Br, I), a linear or branched $C_{1-6}$-alkyl group, a linear or branched $C_{1-6}$ alcoxy group, a formyl group, a hydroxy group, a trifluoromethyl group, a trifluoromethoxy group, a -CO-$C_{1-6}$-alkyl group, a cyano group, a nitro group, a carboxy group, a —CO-O-$C_{1-6}$-alkyl group, a —CO-$NR^AR^B$- moiety, a -CO-NH-$NR^CR^D$-moiety, an -SH, an -S-$C_{1-6}$-alkyl group, an -SO-$C_{1-6}$-alkyl group, an $-SO_2$-$C_{1-6}$-alkyl group, a -$C_{1-6}$-alkylene-S-$C_{1-6}$-alkyl group, a -$C_{1-6}$-alkylene-SO-$C_{1-6}$-alkyl group, a -$C_{1-6}$-alkylene-$SO_2$-$C_{1-6}$-alkyl group, an —$NH_2$-moiety, an NHR'-moiety or an NR'R"-moiety, wherein R' and R" independently represent a linear or branched $C_{1-6}$-alkyl group, a $C_{1-6}$-alkyl group substituted by one or more hydroxy groups and a -$C_{1-6}$-alkylene-$NR^ER^F$ group,
whereby $R^A$, $R^B$, identical or different, represent hydrogen or a $C_{1-6}$-alkyl group, or $R^A$ and $R^B$ together with the bridging nitrogen atom form a saturated, mono- or bicyclic, 3-10 membered heterocyclic ring system, which may be at least mono-substituted by one or more, identical or different, $C_{1-6}$ alkyl groups and/or which may contain at least one further heteroatom selected from the group consisting of nitrogen, oxygen and sulphur as a ring member,
$R^C$, $R^D$, identical or different, represent a hydrogen atom, a $C_{1-6}$-alkyl group, a -CO-O-$C_{1-6}$-alkyl group, a $C_{3-8}$-cycloalkyl group, a $C_{1-6}$-alkylene-$C_{3-8}$-cycloalkyl group, $C_{1-6}$-alkylene-O-$C_{1-6}$-alkyl group or a $C_{1-6}$-alkyl group substituted with one or more hydroxy groups, or $R^C$, $R^D$ together with the bridging nitrogen atom form a saturated, mono- or bicyclic, 3-10 membered heterocyclic ring system, which may be at least mono-substituted by one or more substituents independently selected from the group consisting of $C_{1-6}$ alkyl group, a -CO-$C_{1-6}$-alkyl group, a -CO-O- $C_{1-6}$-alkyl group, a - CO-NH-$C_{1-6}$-alkyl group, a -CS-NH- $C_{1-6}$-alkyl group, an oxo group, a $C_{1-6}$-alkyl group substituted with one or more hydroxy groups, a $C_{1-6}$-alkylene-O-$C_{1-6}$-alkyl group and a —CO-$NH_2$ group and/or which may contain at least one further heteroatom selected from the group consisting of nitrogen, oxygen and sulphur as a ring member, and
wherein $R^E$, $R^F$, identical or different, represent hydrogen or a $C_{1-6}$-alkyl group, or $R^E$ and $R^F$ together with the bridging nitrogen atom form a saturated, mono- or bicyclic, 3-10 membered heterocyclic ring system, which may be at least mono-substituted by one or more, identical or different $C_{1-6}$ alkyl groups and/or which may contain at least one further heteroatom selected from the group consisting of nitrogen, oxygen and sulphur as a ring member.

Preferred aryl groups, which may optionally be at least mono-substituted, are phenyl and naphthyl.

If one or more of the residues $R^3$-$R^8$ represents or comprises a heteroaryl group, which is substituted by one or more, e.g. 1, 2, 3, 4 or 5 substituents, unless defined otherwise, each of the substituents may be independently selected from the group consisting of a halogen atom (e.g. F, Cl, Br, I), a linear or branched $C_{1-6}$-alkyl group, a linear or branched $C_{1-6}$ alcoxy group, a formyl group, a hydroxy group, a trifluoromethyl group, a trifluoromethoxy group, a -CO-$C_{1-6}$-alkyl group, a cyano group, a carboxy group, a -CO-O-$C_{1-6}$-alkyl group, a -CO-$NR^AR^B$- moiety, a -CO-NH-$NR^CR^D$-moiety, an -S-$C_{1-6}$-alkyl group, an -SO-$C_{1-6}$-alkyl group, an $-SO_2$-$C_{1-6}$-alkyl group, a -$C_{1-6}$-alkylene-S-$C_{1-6}$-alkyl group, a -$C_{1-6}$-alkylene-SO-$C_{1-6}$-alkyl group, a -$C_{1-6}$-alkylene-$SO_2$-$C_{1-6}$-alkyl group, a $C_{1-6}$-alkyl group substituted by one or more hydroxy groups and a —$C_{1-6}$-alkylene-$NR^ER^F$ group,
whereby $R^A$, $R^B$, identical or different, represent hydrogen or a $C_{1-6}$-alkyl group, or $R^A$ and $R^B$ together with the bridging nitrogen atom form a saturated, mono- or bicyclic, 3-10 membered heterocyclic ring system, which may be at least mono-substituted by one or more, identical or different, $C_{1-6}$ alkyl groups and/or which may contain at least one further heteroatom selected from the group consisting of nitrogen, oxygen and sulphur as a ring member,

$R^C$, $R^D$, identical or different, represent a hydrogen atom, a $C_{1-6}$-alkyl group, a -CO-O-$C_{1-6}$-alkyl group, a $C_{3-8}$-cycloalkyl group, a $C_{1-6}$-alkylene-$C_{3-8}$-cycloalkyl group, $C_{1-6}$-alkylene-O-$C_{1-6}$-alkyl group or a $C_{1-6}$-alkyl group substituted with one or more hydroxy groups, or $R^C$, $R^D$ together with the bridging nitrogen atom form a saturated, mono- or bicyclic, 3-10 membered heterocyclic ring system, which may be at least mono-substituted by one or more substituents independently selected from the group consisting of $C_{1-6}$ alkyl group, a -CO-$C_{1-6}$-alkyl group, a -CO-O- $C_{1-6}$-alkyl group, a -CO-NH-$C_{1-6}$-alkyl group, a -CS-NH- $C_{1-6}$-alkyl group, an oxo group, a $C_{1-6}$-alkyl group substituted with one or more hydroxy groups, a $C_{1-6}$-alkylene-O-$C_{1-6}$-alkyl group and a —CO-$NH_2$ group and/or which may contain at least one further heteroatom selected from the group consisting of nitrogen, oxygen and sulphur as a ring member, and

wherein $R^E$, $R^F$, identical or different, represent hydrogen or a $C_{1-6}$-alkyl group, or $R^E$ and $R^F$ together with the bridging nitrogen atom form a saturated, mono- or bicyclic, 3-10 membered heterocyclic ring system, which may be at least mono-substituted by one or more, identical or different $C_{1-6}$ alkyl groups and/or which may contain at least one further heteroatom selected from the group consisting of nitrogen, oxygen and sulphur as a ring member,

The heteroatoms, which are present as ring members in the heteroaryl radical, may, unless defined otherwise, independently be selected from the group consisting of nitrogen, oxygen and sulphur. Preferably a heteroaryl radical may comprise 1, 2 or 3 heteroatoms independently selected from the group consisting of N, O and S as ring members.

Suitable heteroaryl groups, which may optionally be at least mono-substituted, may preferably be selected from the group consisting of thienyl, furyl, pyrrolyl, pyridinyl, imidazolyl, pyrimidinyl, pyrazinyl, indolyl, chinolinyl, isochinolinyl, benzo[1,2,5]-thiodiazolyl, benzo[b]thiophenyl, benzo[b]furanyl, imidazo[2,1-b]thiazolyl, triazolyl, and pyrazolyl, more preferably be selected from the group consisting of thienyl-, benzo[1,2,5]-thiodiazolyl, benzo[b]thiophenyl, imidazo [2,1-b]thiazolyl, triazolyl and pyrazolyl.

If one or more of the residues $R^4$-$R^8$ represents or comprises a linear or branched, saturated or unsaturated aliphatic group such as an alkyl group, which is substituted by one or more, e.g. 1, 2, 3, 4 or 5 substituents, unless defined otherwise, each of the substituents may be independently selected from the group consisting of hydroxy, fluorine, chlorine, bromine, branched or unbranched $C_{1-4}$-alkoxy, branched or unbranched $C_{1-4}$-perfluoroalkoxy, branched or unbranched $C_{1-4}$-perfluoroalkyl, amino, carboxy, amido, cyano, nitro, -$SO_2NH_2$, -CO-$C_{1-4}$-alkyl, -SO-$C_{1-4}$-alkyl, -$SO_2$-$C_{1-4}$-alkyl, -NH-$SO_2$-$C_{1-4}$-alkyl, wherein the $C_{1-4}$-alkyl may in each case be branched or unbranched, and a phenyl group, more preferably be selected from the group consisting of hydroxy, F, Cl, Br, methoxy, ethoxy, $CF_3$ and a phenyl group.

Preferred linear or branched, saturated or unsaturated aliphatic groups, which may be substituted by one or more substituents, may preferably be selected from the group consisting of methyl, ethyl, n-propyl, isopropyl, n-butyl, iso-butyl, sec-butyl, tert-butyl, n-pentyl, n-hexyl, n-heptyl, n-octyl, n-nonyl, n-decyl, vinyl, ethinyl, propenyl, propinyl, butenyl and butinyl.

If any of the residues $R^4$-$R^8$ represents or comprises a linear or branched alkylene group, said alkylene group may preferably be selected from the group consisting of - methylene -($CH_2$)-, ethylene -($CH_2$-$CH_2$)-, n-propylene -($CH_2$-$CH_2$-$CH_2$)- or isopropylene -(-$C(CH_3)_2$)-.

Unless otherwise stated, the compounds of the invention are also meant to include compounds which differ only in the presence of one or more isotopically enriched atoms. For example, compounds having the present structures except for the replacement of a hydrogen by a deuterium or tritium, or the replacement of a carbon by $^{13}C$- or $^{14}C$-enriched carbon or $^{15}N$-enriched nitrogen are within the scope of this invention.

Preferred are substituted pyrazoline compounds of general formula I given above, wherein

$R^1$ represents an optionally at least mono-substituted phenyl group,

$R^2$ represents an optionally at least mono-substituted phenyl group,

$R^3$ represents a saturated or unsaturated, optionally at least mono-substituted, optionally at least one heteroatom as ring member containing cycloaliphatic group, which may be condensed with an optionally at least mono-substituted mono- or polycyclic ring system, or $R^3$ represents an optionally at least mono-substituted aryl or heteroaryl group, which may be condensed with an optionally at least mono-substituted mono- or polycyclic ring system, or $R^3$ represents an -$NR^4R^5$-moiety,

$R^4$ and $R^5$, identical or different, represent a hydrogen atom, an unbranched or branched, saturated or unsaturated, optionally at least mono-substituted aliphatic radical, a saturated or unsaturated, optionally at least mono-substituted, optionally at least one heteroatom as ring member containing cycloaliphatic group, which may be condensed with an optionally at least mono-substituted mono- or polycyclic ring system, or an optionally at least mono-substituted aryl or heteroaryl group, which may be condensed with an optionally at least mono-substituted mono- or polycyclic ring system and/or bonded via a linear or branched alkylene group, an -$SO_2$-$R^6$-moiety, or an -$NR^7R^8$-moiety,

$R^6$ represents a linear or branched, saturated or unsaturated, optionally at least mono-substituted aliphatic group, a saturated or unsaturated, optionally at least mono-substituted, optionally at least one heteroatom as ring member containing cycloaliphatic group, which may be condensed with a mono- or polycyclic ring-system, or an optionally at least mono-substituted aryl or heteroaryl group, which may be condensed with a mono- or polycyclic ring system and/or bonded via a linear or branched alkylene group,

$R^7$ and $R^8$, identical or different, represent a hydrogen atom, an unbranched or branched, saturated or unsaturated, optionally at least mono-substituted aliphatic radical, a saturated or unsaturated, optionally at least mono-substituted, optionally at least one heteroatom as ring member containing cycloaliphatic group, which may be condensed with an optionally at least mono-substituted mono- or polycyclic ring system, or an optionally at least mono-substituted aryl or heteroaryl group, which may be condensed with an optionally at least mono-substituted mono- or polycyclic ring system and/or bonded via a linear or branched alkylene group,

optionally in form of a corresponding N-oxide thereof, or a corresponding salt thereof, or a corresponding solvate thereof, whereby the following provisos (disclaimers) may apply, namely
that $R^4$ and $R^5$ do not both represent a hydrogen atom, and/or that if one of the residues $R^4$ and $R^5$ represents a hydrogen atom or a linear or branched, saturated or unsaturated, substituted or unsubstituted aliphatic group, the other one of these residues $R^4$ and $R^5$ does not represent a substituted or unsubstituted pyridyl group, a substituted or unsubstituted pyrimidyl group, a substituted or unsubstituted pyridazyl group, a substituted or unsubstituted pyrazinyl group, a substituted or unsubstituted thienyl group, a substituted or unsubstituted benzyl group, a substituted or unsubstituted phenethyl group, a substituted or unsubstituted phenyl group, a substituted or unsubstituted phenyl group, which is condensed (attached) to at least one, optionally substituted ring or ringsystem, an -NH-phenyl-moiety, wherein the phenyl group may be at least mono-substituted, an unsubstituted or substituted thiazole group, or an unsubstituted or substituted [1,3,4]thiadiazole group.

Preferred are also substituted pyrazoline compounds of general formula I given above, wherein $R^1$ represents a phenyl group, which is optionally substituted by one or more substituents independently selected from the group consisting of a linear or branched $C_{1-6}$-alkyl group, a linear or branched $C_{1-6}$-alkoxy group, a halogen atom, $CH_2F$, $CHF_2$, $CF_3$, CN, OH, $NO_2$, -(C=O)-R', SH, SR', SOR', $SO_2R'$, $NH_2$, NHR', NR'R", -(C=O)-$NH_2$, -(C=O)-NHR' and -(C=O)-NR'R", whereby R' and R" for each substituent independently represent linear or branched $C_{1-6}$ alkyl, preferably $R^1$ represents a phenyl group, which is optionally substituted by one or more substituents selected from the group consisting of methyl, ethyl, F, Cl, Br and $CF_3$, more preferably $R^1$ represents a phenyl group, which is substituted with a chlorine atom in the 4-position, and $R^2$-$R^8$ have the meaning given above, optionally in form of a corresponding N-oxide thereof, or a corresponding salt thereof, or a corresponding solvate thereof.

Also preferred are substituted pyrazoline compounds of general formula I given above, wherein $R^2$ represents a phenyl group, which is optionally substituted by one or more substituents independently selected from the group consisting of a linear or branched $C_{1-6}$-alkyl group, a linear or branched $C_{1-6}$-alkoxy group, a halogen atom, $CH_2F$, $CHF_2$, $CF_3$, CN, OH, $NO_2$, -(C=O)-R', SH, SR', SOR', $SO_2R'$, $NH_2$, NHR', NR'R", -(C=O)-$NH_2$, -(C=O)-NHR' and -(C=O)-NR'R" whereby R' and R" for each substituent independently represent linear or branched $C_{1-6}$ alkyl, preferably $R^2$ represents a phenyl group, which is optionally substituted by one or more substituents selected from the group consisting of methyl, ethyl, F, Cl, Br and $CF_3$, more preferably $R^2$ a phenyl group, which is di-substituted with two chlorine atoms in the 2- and 4-position, and $R^1$ and $R^3$-$R^8$ have the meaning given above, optionally in form of a corresponding N-oxide thereof, or a corresponding salt thereof, or a corresponding solvate thereof.

Preference is also given to substituted pyrazoline compounds of general formula I given above, wherein $R^3$ represents a saturated or unsaturated, optionally at least mono-substituted, optionally at least one heteroatom as ring member containing $C_{3-8}$ cycloaliphatic group, which may be condensed with an optionally at least mono-substituted mono- or polycyclic ring system, or $R^3$ represents an optionally at least mono-substituted, 5- or 6-membered aryl or heteroaryl group, which may be condensed with an optionally at least mono-substituted mono- or polycyclic ring system, or $R^3$ represents an -$NR^4R^5$-moiety, preferably $R^3$ represents a saturated, optionally at least mono-substituted, optionally one or more nitrogen-atoms as ring member containing $C_{3-8}$ cycloaliphatic group, which may be condensed with an optionally at least mono-substituted mono- or polycyclic ring system, or $R^3$ represents an -$NR^4R^5$-moiety, more preferably $R^3$ represents a pyrrolidinyl group, a piperidinyl group or a piperazinyl group, whereby each of these groups may be substituted with one or more $C_{1-6}$-alkyl groups, or $R^3$ represents an -$NR^4R^5$-moiety and $R^1$, $R^2$ and $R^4$-$R^8$ have the meaning given above, optionally in form of a corresponding N-oxide thereof, or a corresponding salt thereof, or a corresponding solvate thereof.

Furthermore, substituted pyrazoline compounds of general formula I given above are preferred, wherein $R^4$ and $R^5$, identical or different, represent a hydrogen atom, an unbranched or branched, saturated or unsaturated, optionally at least mono-substituted $C_{1-6}$-aliphatic radical, a saturated or unsaturated, optionally at least mono-substituted, optionally at least one heteroatom as ring member containing $C_{3-8}$-cycloaliphatic group, which may be condensed with an

optionally at least mono-substituted mono- or polycyclic ring system, or an optionally at least mono-substituted, 5- or 6-membered aryl or heteroaryl group, which may be condensed with an optionally at least mono-substituted mono- or polycyclic ring system and/or bonded via a methylene ($-CH_2-$) or ethylene ($-CH_2-CH_2$)-group, an $-SO_2-R^6$-moiety, or an $-NR^7R^8$-moiety, preferably one of these residues $R^4$ and $R^5$ represents a hydrogen atom and the other one of these residues $R^4$ and $R^5$ represents a saturated or unsaturated, optionally at least mono-substituted, optionally at least one heteroatom as ring member containing $C_{3-8}$-cycloaliphatic group, which may be condensed with an optionally at least mono-substituted mono- or polycyclic ring system, or an optionally at least mono-substituted, 5- or 6-membered aryl or heteroaryl group, which may be condensed with an optionally at least mono-substituted mono- or polycyclic ring system, an —$SO_2-R^6$-moiety, or an $-NR^7R^8$-moiety, or $R^4$ and $R^5$, identical or different, each represent a $C_{1-6}$ alkyl group, more preferably one of these residues $R^4$ and $R^5$ represents a hydrogen atom and the other one of these residues $R^4$ and $R^5$ represents an optionally at least mono-substituted pyrrolidinyl group, an optionally at least mono-substituted piperidinyl group, an optionally at least mono-substituted piperazinyl group, an optionally at least mono-substituted triazolyl group, an $-SO_2-R^6$-moiety, or an $-NR^7R^8$-moiety, or $R^4$ and $R^5$, identical or different, represent a methyl group, an ethyl group, an n-propyl group, an isopropyl group, an n-butyl group, a sec-butyl group or a tert.-butyl group, and $R^1$-$R^3$ and $R^6$-$R^8$ have the meaning given above, optionally in form of a corresponding N-oxide thereof, or a corresponding salt thereof, or a corresponding solvate thereof.

Also preferred are substituted pyrazoline compounds of general formula I given above, wherein $R^6$ represents a linear or branched, saturated or unsaturated, optionally at least mono-substituted $C_{1-6}$ aliphatic group, a saturated or unsaturated, optionally at least mono-substituted, optionally at least one heteroatom as ring member containing $C_{3-8}$ cycloaliphatic group, which may be condensed with a mono- or polycyclic ring-system, or an optionally at least mono-substituted, 5- or 6-membered aryl or heteroaryl group, which may be condensed with a mono- or polycyclic ring system and/or bonded via a methylene ($-CH_2-$) or ethylene ($-CH_2-CH_2$)-group, preferably $R^6$ represents a $C_{1-6}$-alkyl group, a saturated, optionally at least mono-substituted cycloaliphatic group, which may be condensed with a mono- or polycyclic ring-system, or a phenyl group, which is optionally substituted with one or more $C_{1-6}$ alkyl groups, and $R^1$-$R^5$, $R^7$ and $R^8$ have the meaning given above, optionally in form of a corresponding N-oxide thereof, or a corresponding salt thereof, or a corresponding solvate thereof.

Moreover substituted pyrazoline compounds of general formula I given above are preferred, wherein $R^7$ and $R^8$, identical or different, represent a hydrogen atom, an unbranched or branched, saturated or unsaturated, optionally at least mono-substituted $C_{1-6}$ aliphatic radical, a saturated or unsaturated, optionally at least mono-substituted, optionally at least one heteroatom as ring member containing $C_{3-8}$ cycloaliphatic group, which may be condensed with an optionally at least mono-substituted mono- or polycyclic ring system, or an optionally at least mono-substituted, 5- or 6 membered aryl or heteroaryl group, which may be condensed with an optionally at least mono-substituted mono- or polycyclic ring system and/or bonded via a methylene ($-CH_2-$) or ethylene ($-CH_2-CH_2$)-group, preferably represent a hydrogen atom or a $C_{1-6}$ alkyl radical, and $R^1$-$R^6$ have the meaning given above, optionally in form of a corresponding N-oxide thereof, or a corresponding salt thereof, or a corresponding solvate thereof.

Particularly preferred are substituted pyrazoline compounds of general formula I

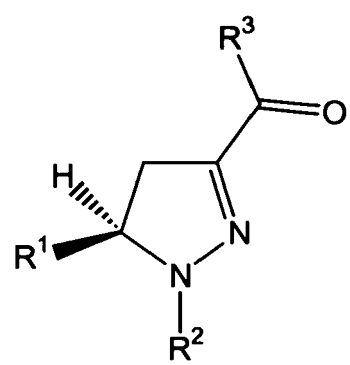

I

wherein

$R^1$ represents a phenyl group, which is optionally substituted by one or more substituents independently selected from the group consisting of methyl, ethyl, F, Cl, Br and $CF_3$,

$R^2$ represents a phenyl group, which is optionally substituted by one or more substituents independently selected from the group consisting of methyl, ethyl, F, Cl, Br and $CF_3$,

$R^3$ represents a pyrrolidinyl group, a piperidinyl group or a piperazinyl group, whereby each of these groups may be substituted with one or more $C_{1-6}$-alkyl groups, or $R^3$ represents an —$NR^4R^5$-moiety,

one of the residues $R^4$ and $R^5$ represents a hydrogen atom and the other one of these residues $R^4$ and $R^5$ represents an optionally at least mono-substituted pyrrolidinyl group; an optionally at least mono-substituted piperidinyl group; an optionally at least mono-substituted piperazinyl group; an optionally at least mono-substituted triazolyl group; an —$SO_2$-$R^6$-moiety; or an -$NR^7R^8$-moiety, or $R^4$ and $R^5$, identical or different, represent a methyl group, an ethyl group, an n-propyl group, an isopropyl group, an n-butyl group, a sec-butyl group or a tert.-butyl group,

$R^6$ represents a $C_{1-6}$-alkyl group; a saturated, optionally at least mono- substituted cycloaliphatic group, which may be condensed with a mono- or polycyclic ring- system; or a phenyl group, which is optionally substituted with one or more $C_{1-6}$ alkyl groups, and

and $R^8$, identical or different, represent a hydrogen atom or a $C_{1-6}$ alkyl radical

optionally in form of a corresponding N-oxide thereof, or a corresponding salt thereof, or a corresponding solvate thereof.

Also particularly preferred are substituted pyrazoline compounds of general formula I

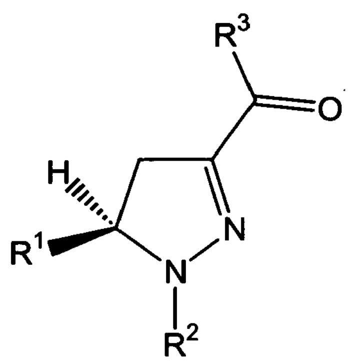

I

wherein

$R^1$ represents a phenyl group, which is optionally substituted with 1, 2, 3, 4 or 5 substituents independently selected from the group consisting of linear or branched $C_{1-6}$-alkyl, linear or branched $C_{1-6}$-alkoxy, F, Cl, Br, I, $CH_2F$, $CHF_2$, $CF_3$, CN, OH, $NO_2$, -(C=O)-R', SH, SR', SOR', $SO_2R'$, $NH_2$, NHR', NR'R", -(C=O)-$NH_2$, -(C=O)-NHR' and —(C=O)-NR'R", whereby R' and R" at each ocurrence independently represent a linear or branched $C_{1-6}$ alkyl group,

$R^2$ represents a phenyl group, which is optionally substituted with 1, 2, 3, 4 or 5 substituents independently selected from the group consisting of linear or branched $C_{1-6}$-alkyl, linear or branched $C_{1-6}$-alkoxy, F, Cl, Br, I, $CH_2F$, $CHF_2$, $CF_3$, CN, OH, $NO_2$, -(C=O)-R', SH, SR', SOR', $SO_2R'$, $NH_2$, NHR', NR'R", -(C=O)-$NH_2$, -(C=O)-NHR' and —(C=O)-NR'R", whereby R' and R" at each ocurrence independently represent a linear or branched $C_{1-6}$ alkyl group,

$R^3$ represents a saturated or unsaturated $C_{3-8}$ cycloaliphatic group, whereby said $C_{3-8}$ cycloaliphatic group is optionally substituted with 1, 2, 3 or 4 substituents independently selected from the group consisting of linear or branched $C_{1-6}$ alkyl, linear or branched $C_{1-6}$ alkoxy, OH, F, Cl, Br, I, CN, $CH_2F$, $CHF_2$, $CF_3$ and oxo (=O) and whereby said $C_{3-8}$ cycloaliphatic group may contain 1, 2 or 3 heteroatoms independently selected from the group consisting of N, O and S as ring members, or $R^3$ represents an -$NR^4R^5$-moiety,

$R^4$ represents a hydrogen atom or a linear or branched $C_{1-6}$-alkyl group,

$R^5$ represents a linear or branched $C_{1-6}$ alkyl group; an $-SO_2-R^6$-moiety; a saturated or unsaturated $C_{3-8}$ cycloaliphatic group, whereby said $C_{3-8}$ cycloaliphatic group is optionally substituted with 1, 2, 3 or 4 substituents independently selected from the group consisting of linear or branched $C_{1-6}$ alkyl group, a linear or branched $C_{1-6}$ alkoxy group, OH, F, Cl, Br, I, CN $CH_2F$, $CHF_2$, $CF_3$ and oxo (=O) and whereby said $C_{3-8}$ cycloaliphatic group may contain 1, 2 or 3 heteroatoms independently selected from the group consisting of N, O and S as ring members, and

$R^6$ represents a phenyl group, which is optionally substituted with 1, 2, 3, 4 or 5 substituents independently selected from the group consisting of a linear or branched $C_{1-6}$-alkyl group, a linear or branched $C_{1-6}$-alkoxy group, F, Cl, Br, I, $CH_2F$, $CHF_2$, $CF_3$, CN, OH, $NO_2$, -(C=O)-R', SH, SR', SOR', $SO_2R'$, $NH_2$, NHR', NR'R'', -(C=O)-$NH_2$, -(C=O)-NHR' and -(C=O)-NR'R'', whereby R' and R'' at each ocurrence independently represent a linear or branched $C_{1-6}$ alkyl group,

optionally in form of a corresponding N-oxide thereof, or a corresponding salt thereof, or a corresponding solvate thereof.

Also particularly preferred are substituted pyrazoline compounds of general formula I

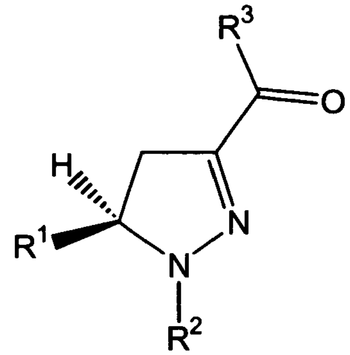

I

wherein

$R^1$ represents a phenyl group, which is optionally substituted with 1, 2, 3, 4 or 5 substituents independently selected from the group consisting of methyl, ethyl, F, Cl, Br and $CF_3$,

$R^2$ represents a phenyl group, which is optionally substituted with 1, 2, 3, 4 or 5 substituents independently selected from the group consisting of methyl, ethyl, F, Cl, Br and $CF_3$,

$R^3$ represents a pyrrolidinyl group, a piperidinyl group or a piperazinyl group, whereby each of these groups may be substituted with one or more of $C_{1-6}$-alkyl groups, or $R^3$ represents an $-NR^4R^5$-moiety,

$R^4$ represents a hydrogen atom or a linear or branched $C_{1-6}$-alkyl group,

$R^5$ represents a linear or branched $C_{1-6}$ alkyl group; an $-SO_2-R^6$-moiety; a pyrrolidinyl group; a piperidinyl group; a piperazinyl group; a homo-piperazinyl group; a morpholinyl group; a triazolyl group; whereby each of the heterocyclic rings may be substituted with one or more, identical or different, $C_{1-6}$-alkyl groups, and

$R^6$ represents a phenyl group, which is optionally substituted with one or more $C_{1-6}$ alkyl groups, which may be identical or different,

optionally in form of a corresponding N-oxide thereof, or a corresponding salt thereof, or a corresponding solvate thereof.

Also particularly preferred are substituted pyrazoline compounds of general formula I

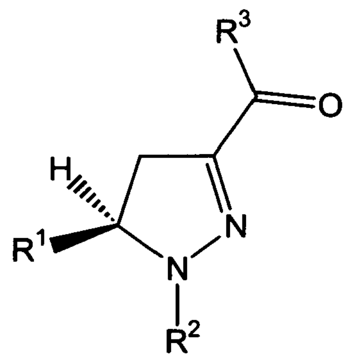

I

wherein

$R^1$ represents a phenyl ring, which is mono-substituted with a halogen atom, preferably a chlorine atom, in its 4-position,

$R^2$ represents a phenyl ring, which is di-substituted with two halogen atoms, preferably chlorine atoms, in its 2- and 4-position,

$R^3$ represents a pyrrolidinyl group, a piperidinyl group, a piperazinyl group, a homo-piperazinyl group, a morpholinyl group, or an $-NR^4R^5$-moiety,

$R^4$ represents a hydrogen atom or a linear or branched $C_{1-6}$-alkyl group,

$R^5$ represents a linear or branched $C_{1-6}$ alkyl group; an $-SO_2-R^6$-moiety; a pyrrolidinyl group; a piperidinyl group; a piperazinyl group; a homo-piperazinyl group; a morpholinyl group; or a triazolyl group whereby each of the heterocyclic rings may be substituted with one or more, identical or different, $C_{1-6}$-alkyl groups, and

$R^6$ represents a phenyl group, which is optionally substituted with one or more $C_{1-6}$ alkyl groups, which may be identical or different,

optionally in form of a corresponding N-oxide thereof, or a corresponding salt thereof, or a corresponding solvate thereof.

Most particularly preferred is the compound (R)-N-piperidinyl-5-(4-chloro-phenyl)-1-(2,4-dichlorophenyl)-4,5-dihydro-1H-pyrazol-3-carboxamide:

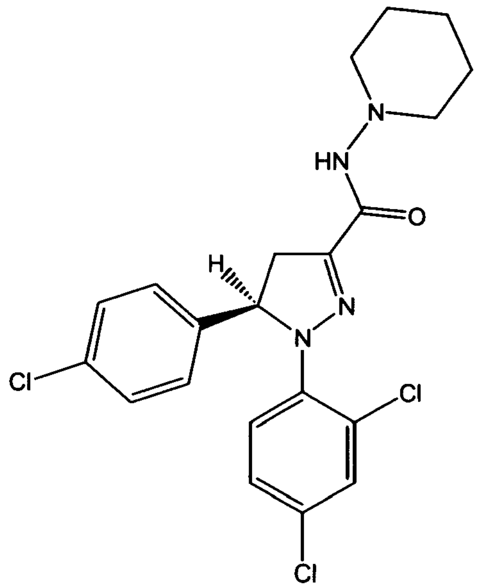

optionally in the form of a corresponding N-oxide, a corresponding salt or a corresponding solvate.

In another aspect the present invention also provides a process for the preparation of substituted pyrazoline compounds of general formula I given above, according to which at least one compound of general formula IIa

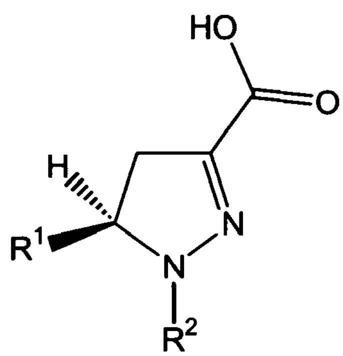

**(IIa)**

wherein $R^1$ and $R^2$ have the meaning given above, is optionally transferred under inert atmosphere to a compound of general formula (III) via reaction with an activating agent

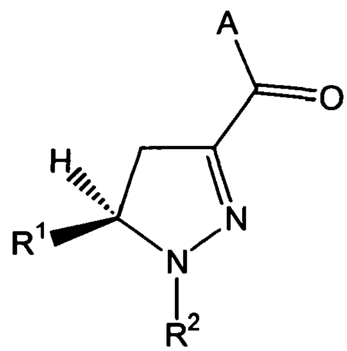

(III)

wherein the substituents $R^1$ and $R^2$ have the meaning given above and A represents a leaving group, preferably a chlorine atom, said compound being optionally isolated and/or optionally purified, and at least one compound of general formula (IIa) is reacted with a compound of general formula $R^3H$, wherein $R^3$ represents an $-NR^4R^5$-moiety, with $R^4$ and $R^5$ having the meaning given above, under inert atmosphere to yield a substituted pyrazoline compound of general formula I, wherein $R^3$ represents an $-NR^4R^5$-moiety, or at least one compound of general formula (III) is reacted with a compound of the general formula $R^3H$, in which $R^3$ has the meaning given above under inert atmosphere to yield a compound of general formula (I), which is optionally isolated and/or optionally purified.

Preferably the compound IIa is obtained from a mixture comprising the enantiomers

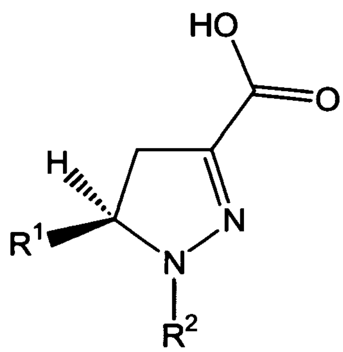

and

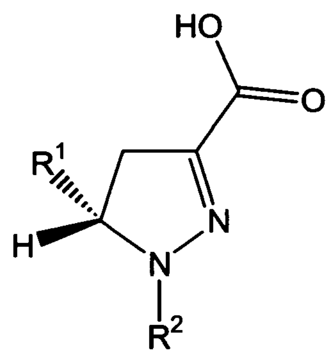

(IIa) (IIb).

The compound (IIa) may be obtained from the mixture of the enantiomers by means well known to those skilled in the art. Preferably, the compound IIa is obtained from the mixture in form of an addition compound with a chiral base, preferably (+)-Cinchonine or R-(+)-1-Phenylethylamine, and preferably liberated from the addition compound.

Preferably the mixture comprising the enantiomers IIa and IIb may be obtained by reacting at least one benzaldehyde compound of general formula (IV)

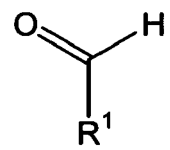

(IV)

wherein $R^1$ has the meaning given above, is reacted with a pyruvate compound of general formula (V)

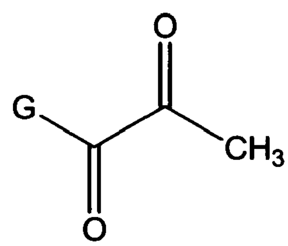

(V),

wherein G represents an OR group with R being a branched or unbranched $C_{1-6}$ alkyl radical, preferably an ethyl radical, or G represents an O-K group with K being a cation, preferably a monovalent cation, more preferably an alkali metal cation, even more preferably a sodium cation, to yield a compound of general formula (VI)

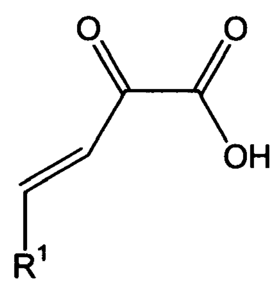

(VI)

wherein $R^1$ has the meaning given above, which is optionally isolated and/or optionally purified, and which is reacted with an optionally substituted phenyl hydrazine of general formula (VII)

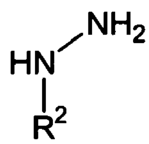

(VII)

or a corresponding salt thereof, wherein $R^2$ has the meaning given above, under inert atmosphere, to yield a mixture of compounds

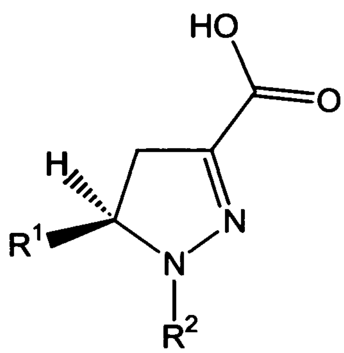

and

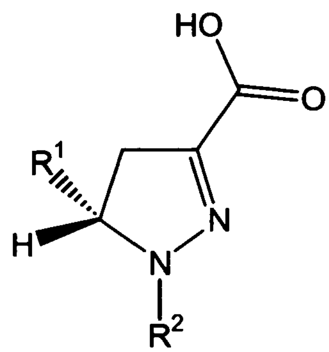

(IIa) (IIb).

The inventive process is also illustrated in scheme I given below:

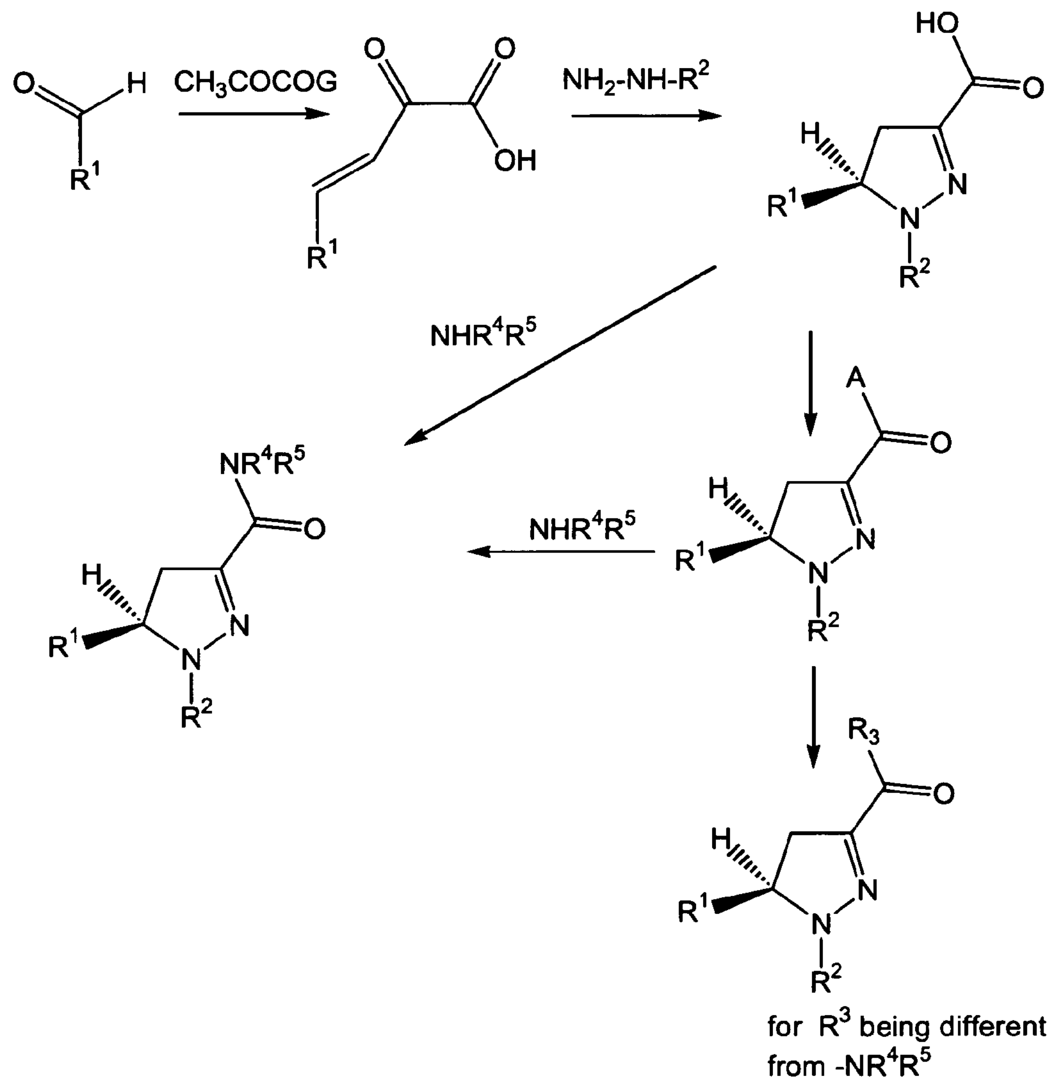


The reaction of the benzaldehyde compound of general formula IV with a pyruvate compound of general formula V is preferably carried out in the presence of at least one base, more preferably in the presence of an alkali metal hydroxide such as sodium hydroxide or potassium hydroxide or an alkali metal methoxide such as sodium methoxide, as described, for example, in Synthetic communications, 26(11), 2229-33, (1996). The respective description is hereby incorporated by reference and forms part of the disclosure. Preferably sodium pyruvate may be used as the pyruvate compound. Preferably said reaction is carried out in a protic reaction medium such as a $C_{1-4}$ alkyl alcohol or mixtures of these. Mixtures of such alcohols with water, e.g. ethanol/water may also be used.

Reaction temperature as well as the duration of the reaction may vary over a broad range. Preferred reaction temperatures range from -10 °C to the boiling point of the reaction medium. Suitable reaction times may vary for example from several minutes to several hours.

Also preferred the reaction of the benzaldehyde compound of general formula IV with a pyruvate compound of general formula V is carried out under acid catalysed conditions, more preferably by refluxing the mixture in dichloromethane in the presence of copper(II)trifluoromethanesulfonate as described, for example, in Synlett, (1), 147-149, 2001. The respective description is hereby incorporated by reference and forms part of the disclosure.

The reaction of the compound of general formula (VI) with an optionally substituted phenyl hydrazin of general formula (VII) is preferably carried out in a suitable reaction medium such as $C_{1-4}$-alcohols or ethers such as dioxane or tetrahydrofurane or mixtures of at least two of these afore mentioned compounds. Also preferably, said reaction may be carried out in the presence of an acid, whereby the acid may be organic such as acetic acid and/or inorganic such as hydrochloric acid. Furthermore, the reaction may also be carried out in the presence of a base such as piperidine, piperazine, sodium hydroxide, potassium hydroxide, sodium methoxide or sodium ethoxide, or a mixture of at least two

of these bases may also be used.

Reaction temperature as well as the duration of the reaction may vary over a broad range. Suitable reaction temperatures range from room temperature, i.e. approximately 25 ˚C to the boiling point of the reaction medium. Suitable reaction times may vary for example from several minutes to several hours.

The carboxylic group of the compound of general formula (III) may be activated for further reactions by the introduction of a suitable leaving group according to conventional methods well known to those skilled in the art. Preferably the compounds of general formula (VI) are transferred into an acid chloride, an acid anhydride, a mixed anhydride, a $C_{1-4}$ alkyl ester, an activated ester such as p-nitrophenylester. Other well known methods for the activation of acids include the activation with N,N-dicyclohexylcarbodiimide or benzotriazol-N-oxotris(dimethylamino) phosphonium hexafluorophosphate (BOP)).

If said activated compound of general formula (III) is an acid chloride, it is preferably prepared by reaction of the corresponding acid of general formula (IIa) with thionyl chloride or oxalyl chloride, whereby said chlorinating agent is also used as the solvent. Also preferably an additional solvent may be used. Suitable solvents include hydrocarbons such as benzene, toluene or xylene, halogenated hydrocarbons such as dichloromethane, chloroform or carbon tetrachloride, ethers such as diethyl ether, dioxane, tetrahydrofurane or dimethoxyethane. Mixtures of two or more solvents from one class or two or more solvents from different classes may also be used. Preferred reaction temperature range from 0˚ C to the boiling point of the solvent and reaction times from several minutes to several hours.

If said activated compound of general formula (III) is a mixed anhydride, said anhydride may preferably be prepared, for example, by reaction of the corresponding acid of general formula (IIa) with ethyl chloroformiate in the presence of a base such as triethylamine or pyridine, in a suitable solvent.

The reaction of general formula (IIa) with a compound of general formula $HR^3$ to yield compounds of general general I, wherein $R^3$ represents an $-NR^4R^5$ moiety is preferably carried out in presence of a base such as triethylamine in a reaction medium such as methylenchloride. The temperature is preferably in the range from 0˚C to the boiling point of the reaction medium. The reaction time may vary over a broad range, e.g. from several hours to several days.

The reaction of general formula (IIa) with a compound of general formula $HR^3$ to yield compounds of general formula I, wherein $R^3$ represents a saturated or unsaturated, optionally at least mono-substituted, optionally at least one heteroatom as ring member containing cycloaliphatic group, which may be condensed with an optionally at least mono-substituted mono- or polycyclic ring system, or an optionally at least mono-substituted aryl or heteroaryl group, which may be condensed with an optionally at least mono-substituted mono- or polycyclic ring system may be carried out according to conventional methods well known to those skilled in the art, e.g. from Pascual, A., J. Prakt Chem., 1999, 341(7), 695-700; Lin, S. et al., Heterocycles, 2001, 55(2), 265-277; Rao, P. et al., J. Org. Chem., 2000, 65(22), 7323-7344, Pearson D.E and Buehler, C.A., Synthesis, 1972, 533-542 and references cited therein. The respective descriptions are hereby incorporated by reference and form part of the present disclosure.

Preferably said reaction is carried out in the presence of a Lewis acid, which is preferably selected from the group consisting of $FeCl_3$, $ZnCl_2$ and $AlCl_3$, in a suitable reaction medium such as toluene, benzene, tetrahydrofurane or similar. The temperature is preferably in teh range from 0˚C to the boiling point of the reaction medium, more preferably from 15 to 25 ˚C. The reaction time may vary over a broad range, e.g. from several minutes to several hours.

The afore mentioned reactions involving the synthesis of the 4,5-dihydro-pyrazole ring or the reaction of a compound comprising said ring are carried out under an inert atmosphere, preferably nitrogen or argon, to avoid oxidation of the ring-system.

During the processes described above the protection of sensitive groups or of reagents may be necessary and/or desirable. The introduction of conventional protective groups as well as their removal may be performed by methods well-known to those skilled in the art.

In a further aspect the present invention also provides a process for the preparation of salts of substituted pyrazoline compounds of general formula (I), wherein at least one compound of general formula (I) having at least one basic group is reacted with at least one inorganic and/or organic acid, preferably in the presence of a suitable reaction medium. Suitable reaction media include, for example, any of the ones given above. Suitable inorganic acids include hydrochloric acid, hydrobromic acid, phosphoric acid, sulfuric acid, nitric acid, suitable organic acids are e.g. citric acid, maleic acid, fumaric acid, tartaric acid, or derivatives thereof, p-toluenesulfonic acid, methanesulfonic acid or camphersulfonic acid.

In yet a further aspect the present invention also provides a process for the preparation of salts of substituted pyrazoline compounds of general formula (I), wherein at least one compound of general formula (I) having at least one acidic group is reacted with one or more suitable bases, preferably in the presence of a suitable reaction medium. Suitable bases are e.g. hydroxides, carbonates or alkoxides, which include suitable cations, derived e.g. from alkaline metals, alkaline earth metals or organic cations, e.g. $[NH_nR_{4-n}]^+$, wherein n is 0, 1, 2, 3 or 4 and R represents a branched or unbranched $C_{1-4}$-alkyl-radical. Suitable reaction media are, for example, any of the ones given above.

The term "salt" is to be understood as meaning any form of the active compound used according to the invention in which it assumes an ionic form or is charged and is coupled with a counter-ion (a cation or anion) or is in solution. By

this are also to be understood complexes of the active compound with other molecules and ions, in particular complexes which are complexed via ionic interactions. Especially this covers any physiologically acceptable salt.

Solvates, preferably hydrates, of the substituted pyrazoline compounds of general formula (I), of corresponding N-oxides or of corresponding salts thereof may also be obtained by standard procedures known to those skilled in the art.

Substituted pyrazoline compounds of general formula I, which comprise nitrogen-atom containing saturated, unsaturated or aromatic rings may also be obtained in the form of their N-oxides by methods well known to those skilled in the art.

Those skilled in the art understand that the term substituted pyrazoline compounds as used herein is to be understood as encompassing derivatives such as ethers, esters and complexes of these compounds as well. The term "derivatives" as used in this application is defined here as meaning a chemical compound having undergone a chemical derivation starting from an acting (active) compound to change (ameliorate for pharmaceutical use) any of its physico-chemical properties, especially a so-called prodrug, e.g. their esters and ethers. Examples of well known methods of producing a prodrug of a given acting compound are known to those skilled in the art and can be found e.g. in Krogsgaard-Larsen et al., Textbook of Drugdesign and Discovery, Taylor & Francis (April 2002). The respective description is hereby incorporated by reference and forms part of the disclosure.

Those skilled in the art understand that substituents of the inventive compounds, particulary substituent $R^3$, may lead to stereoisomers, which are also covered by the present invention.

The purification and isolation of the inventive substituted pyrazoline compounds of general formula (I) and its intermediates, or a salt thereof, or an N-oxide thereof, or a solvate or any intermediate thereof may, if required, be carried out by conventional methods known to those skilled in the art, e.g. chromatographic methods or recrystallization.

The substituted pyrazoline compounds of general formula (I) given below, corresponding N-oxides, corresponding salts thereof and corresponding solvates are toxicologically acceptable and are therefore suitable as pharmaceutical active substances for the preparation of medicaments.

It has been found that the substituted pyrazoline compounds of general formula I given below, N-oxides thereof, corresponding salts and corresponding solvates have a high affinity to cannabinoid receptors, particularly cannabinoid 1 ($CB_1$)-receptors, i.e. they are selective ligands for the ($CB_1$)-receptor and act as modulators, e.g. antagonists, inverse agonists or agonists, on these receptors. In particular, these pyrazoline compounds show little or no development of tolerance during treatment, particularly with respect to food intake, i.e. if the treatment is interrupted for a given period of time and then continued afterwards, the inventively used pyrazoline compounds will again show the desired effect. After ending the treatment with the pyrazoline compounds, the positive influence on the body weight is found to continue.

Furthermore, these pyrazoline compounds show relatively weak Herg channel affinity, thus a low risk of prolongation of the QT-interval is to be expected for these compounds.

In summary, the inventively used pyrazoline compounds are distinguished by a broad spectrum of beneficial effects, while at the same time showing relatively little undesired effects, i.e. effects which do not positively contribute to or even interfere with the well being of the patient.

Thus, an other aspect of the present invention relates to a medicament comprising at least one substituted pyrazoline compound of general formula I, optionally in form of a corresponding N-oxide thereof, or a corresponding salt thereof, or a corresponding solvate thereof, and optionally one or more pharmaceutically acceptable excipients. It is preferred that the inventive compounds are present in the medicament with an enantiomeric excess with respect to their other enantiomer (resulting from the stereocenter at the 5-position) of at least 90 %, more preferably at least 95 %, yet more preferably of at least 99 %. Most preferably, the inventive medicament comprises the substituted pyrazoline compound of general formula I in pure form, i.e. essentially free from its other enantiomer.

The inventive medicament may preferably also comprise any of the inventive pyrazoline compounds or combinations of at least two of these pyrazoline compounds given above.

Said medicament may also comprise any combination of one or more of the substituted pyrazoline compounds of general formula I given above, corresponding N-oxides thereof, physiologically acceptable salts thereof or physiologically acceptable solvates thereof.

Preferably said medicament is suitable for the modulation (regulation) of cannabinoid-receptors, preferably cannabinoid 1 ($CB_1$) receptors, for the prophylaxis and/or treatment of disorders of the central nervous system, disorders of the immune system, disorders of the cardiovascular system, disorders of the endocrinous system, disorders of the respiratory system, disorders of the gastrointestinal tract or reproductive disorders.

Particularly preferably said medicament is suitable for the prophylaxis and/or treatment of psychosis.

Also particularly preferably said medicament is suitable for the prophylaxis and/or treatment of food intake disorders, preferably bulimia, anorexia, cachexia, obesity and/or type II diabetus mellitus (non-insuline dependent diabetes mellitus), more preferably obesity. The inventive medicament also seems to be active in the prophylaxis and/or treatment of appetency disorders, e.g. the pyrazoline compounds of general formula I also reduce the desire for sweets.

Also particularly preferably said medicament is suitable for the prophylaxis and/or treatment of cancer, preferably for the prophylaxis and/or treatment of one or more types of cancer selected from the group consisting of brain cancer,

bone cancer, lip cancer, mouth cancer, esophageal cancer, stomach cancer, liver cancer, bladder cancer, pancreas cancer, ovary cancer, cervical cancer, lung cancer, breast cancer, skin cancer, colon cancer, bowel cancer and prostate cancer, more preferably for the prophylaxis and/or treatment of one or more types of cancer selected from the group consisting of colon cancer, bowel cancer and prostate cancer.

Particularly preferably said medicament is suitable for the prophylaxis and/or treatment of alcohol abuse and/or alcohol addiction, nicotine abuse and/or nicotine addiction, drug abuse and/or drug addiction and/or medicament abuse and/or medicament addiction, preferably drug abuse and/or drug addiction and/or nicotine abuse and/or nicotine addiction.

Medicaments and/or drugs, which are frequently the subject of misuse include opioids, barbiturates, cannabis, cocaine, amphetamines, phencyclidine, hallucinogens and benzodiazepines.

The medicament is also suitable for the prophylaxis and/or treatment of one or more disorders selected from the group consisting of bone disorders, preferably osteoporosis (e.g. osteoporosis associated with a genetic predisposition, sex hormone deficiency, or ageing), cancer-associated bone disease or Paget's disease of bone; schizophrenia, anxiety, depression, epilepsy, neurodegenerative disorders, cerebellar disorders, spinocerebellar disorders, cognitive disorders, cranial trauma, head trauma, stroke, panic attacks, peripheric neuropathy, inflammation, glaucoma, migraine, Morbus Parkinson, Morbus Huntington, Morbus Alzheimer, Raynaud's disease, tremblement disorders, compulsive disorders, senile dementia, thymic disorders, tardive dyskinesia, bipolar disorders, medicament-induced movement disorders, dystonia, endotoxemic shock, hemorragic shock, hypotension, insomnia, immunologic disorders, sclerotic plaques, vomiting, diarrhea, asthma, memory disorders, pruritus, pain, or for potentiation of the analgesic effect of narcotic and non-narcotic analgesics, or for influencing intestinal transit.

Another aspect of the present invention is the use of at least one substituted pyrazoline compound of general formula I given above as suitable active substances, optionally in form of a corresponding N-oxide thereof, or a corresponding salt thereof, or a corresponding solvate thereof, and optionally one or more pharmaceutically acceptable excipients, for the preparation of a medicament for the modulation of cannabinoid-receptors, preferably cannabinoid 1 ($CB_1$) receptors, for the prophylaxis and/or treatment of disorders of the central nervous system, disorders of the immune system, disorders of the cardiovascular system, disorders of the endocrinous system, disorders of the respiratory system, disorders of the gastrointestinal tract or reproductive disorders.

Particularly preferred is the use of at least one of the respective pyrazoline compounds, optionally in form of a corresponding N-oxide thereof, or a corresponding salt thereof, or a corresponding solvate thereof, and optionally one or more pharmaceutically acceptable excipients, for the preparation of a medicament for the prophylaxis and/or treatment of psychosis.

Also particularly preferred is the use of at least one of the respective pyrazoline compounds, optionally in form of a corresponding N-oxide thereof, or a corresponding salt thereof, or a corresponding solvate thereof, and optionally one or more pharmaceutically acceptable excipients, for the preparation of a medicament for the prophylaxis and/or treatment of food intake disorders, preferably bulimia, anorexia, cachexia, obesity and/or type II diabetus mellitus (non-insuline dependent diabetes mellitus), more preferably obesity.

Also particularly preferred is the use of at least one of the pyrazoline compounds as defined herein and optionally one or more pharmaceutically acceptable excipients, for the preparation of a medicament for the treatment of metabolic syndrome.

The metabolic syndrome and definitions thereof are described in detail by Eckel et al., The Lancet, Vol. 365 (2005), 1415-1428, included herewith by reference. One of the respective definitions was established by the WHO in 1998 (as described in Alberti et al., Diabet. Med. 1998, 15, pages 539-53, the respective description thereof is herewith incorporated by reference and forms part of the present disclosure). The other, more widely accepted, definition of the metabolic syndrome was established by the Adult Treatment Panel (ATP III) of the US National Cholesterol Education Program (NCEP) in 2001, as described in JAMA 2001; 285; 2486-97, the respective description thereof is herewith incorporated by reference and forms part of the present disclosure.

The metabolic syndrome is characterized by an interaction of several physiological parameters such as triglycerides, lipids, blood pressure, glucose levels and insuline levels.

Even though obesity may play a critical role in the development of metabolic syndrome, many of its aspects are weight independent, especially some lipid parameters. Especially the positive influence on the weight independent aspects of the metabolic syndrome (see e.g. Pagotto and Pasquali, The Lancet, Vol. 365 (2005), 1363, 1364, included herewith by reference) like some blood parameters, especially lipid parameters is one of the major and surprising advantages of the inventively used substituted pyrazoline compounds.

Another aspect of the invention is the use of one or more pyrazoline compounds as defined herein for the manufacture of a medicament for improvent of cardiovascular and/or metabolic risk factors, such as one or more of the following factors:

Elevated triglycerides, whereby elevated levels of triglycerides are preferably understood as being > 150 mg/dl,

Low HDL cholesterol, whereby low levels of HDL cholesterol are preferably understood as being < 40 mg/dl in men and < 50 mg/dl in women,

Hypertension, whereby hypertension is preferably understood as being > 130/85 mmHg,

Impaired fasting glucose, whereby impaired fasting glucose levels are preferably understood as being > 110 mg/dl,

Insulin resistance

Dyslipidemia.

Another aspect of the invention is the use of one or more pyrazoline compounds as defined herein for the manufacture of a medicament for the treatment of the weight independent aspects of metabolic syndrome.

Another aspect of the invention is a method for improving cardiovascular and/or metabolic risk factors, such as one or more of the following factors:

Elevated triglycerides, whereby elevated levels of triglycerides are preferably understood as being > 150 mg/dl,

Low HDL cholesterol, whereby low levels of HDL cholesterol are preferably understood as being < 40 mg/dl in men and < 50 mg/dl in women,

Hypertension, whereby hypertension is preferably understood as being > 130/85 mmHg,

Impaired fasting glucose, whereby impaired fasting glucose levels are preferably understood as being > 110 mg/dl,

Insulin resistance

Dyslipidemia,

in a subject, preferably a human.

Another aspect of the invention is a method for treating of the weight independent aspects of metabolic syndrome.

Also particularly preferred is the use of at least one of the respective pyrazoline compounds, optionally in form of a corresponding N-oxide thereof, or a corresponding salt thereof, or a corresponding solvate thereof, and optionally one or more pharmaceutically acceptable excipients, for the preparation of a medicament for the prophylaxis and/or treatment of cancer, preferably for the prophylaxis and/or treatment of one or more types of cancer selected from the group consisting of brain cancer, bone cancer, lip cancer, mouth cancer, esophageal cancer, stomach cancer, liver cancer, bladder cancer, pancreas cancer, ovary cancer, cervical cancer, lung cancer, breast cancer, skin cancer, colon cancer, bowel cancer and prostate cancer, more preferably for the prophylaxis and/or treatment of one or more types of cancer selected from the group consisting of colon cancer, bowel cancer and prostate cancer.

Also particularly preferred is the use of at least one of the respective pyrazoline compounds, optionally in form of a corresponding N-oxide thereof, or a corresponding salt thereof, or a corresponding solvate thereof, and optionally one or more pharmaceutically acceptable excipients, for the preparation of a medicament for the prophylaxis and/or treatment of alcohol abuse and/or alcohol addiction, nicotine abuse and/or nicotine addiction, drug abuse and/or drug addiction and/or medicament abuse and/or medicament addiction, preferably drug abuse and/or drug addiction and/or nicotine abuse and/or nicotine addiction.

Medicaments/drugs, which are frequently the subject of misuse include opioids, barbiturates, cannabis, cocaine, amphetamines, phencyclidine, hallucinogens and benzodiazepines.

Also preferred is the use of at least one of the respective pyrazoline compounds, optionally in form of a corresponding N-oxide thereof, or a corresponding salt thereof, or a corresponding solvate thereof, and optionally one or more pharmaceutically acceptable excipients, for the preparation of a medicament for the prophylaxis and/or treatment of one or more disorders selected from the group consisting of bone disorders, preferably osteoporosis (e.g. osteoporosis associated with a genetic predisposition, sex hormone deficiency, or ageing), cancer-associated bone disease or Paget's disease of bone; schizophrenia, anxiety, depression, epilepsy, neurodegenerative disorders, cerebellar disorders, spinocerebellar disorders, cognitive disorders, cranial trauma, head trauma, stroke, panic attacks, peripheric neuropathy, inflammation, glaucoma, migraine, Morbus Parkinson, Morbus Huntington, Morbus Alzheimer, Raynaud's disease, tremblement disorders, compulsive disorders, senile dementia, thymic disorders, tardive dyskinesia, bipolar disorders, ,

medicament-induced movement disorders, dystonia, endotoxemic shock, hemorragic shock, hypotension, insomnia, immunologic disorders, sclerotic plaques, vomiting, diarrhea, asthma, memory disorders, pruritus, pain, or for potentiation of the analgesic effect of narcotic and non-narcotic analgesics, or for influencing intestinal transit.

The medicament according to the present invention may be in any form suitable for the application to humans and/or animals, preferably humans including infants, children and adults and can be produced by standard procedures known to those skilled in the art. The composition of the medicament may vary depending on the route of administration.

The medicament of the present invention may for example be administered parentally in combination with conventional injectable liquid carriers, such as water or suitable alcohols. Conventional pharmaceutical excipients for injection, such as stabilizing agents, solubilizing agents, and buffers, may be included in such injectable compositions. These medicaments may for example be injected intramuscularly, intraperitoneally, or intravenously.

Medicaments according to the present invention may also be formulated into orally administrable compositions containing one or more physiologically compatible carriers or excipients, in solid or liquid form. These compositions may contain conventional ingredients such as binding agents, fillers, lubricants, and acceptable wetting agents. The compositions may take any convenient form, such as tablets, pellets, capsules, lozenges, aqueous or oily solutions, suspensions, emulsions, or dry powdered forms suitable for reconstitution with water or other suitable liquid medium before use, for immediate or retarded release.

The liquid oral forms for administration may also contain certain additives such as sweeteners, flavoring, preservatives, and emulsifying agents. Non-aqueous liquid compositions for oral administration may also be formulated, containing edible oils. Such liquid compositions may be conveniently encapsulated in e.g., gelatin capsules in a unit dosage amount.

The compositions of the present invention may also be administered topically or via a suppository.

The daily dosage for humans and animals may vary depending on factors that have their basis in the respective species or other factors, such as age, sex, weight or degree of illness and so forth. The daily dosage for humans may preferably be in the range from1 to 2000, preferably 1 to 1500, more preferably 1 to 1000 milligrams of active substance to be administered during one or several intakes per day.

**Pharmacological Methods:**

**I. In-vitro determination of affinity to CB1/CB2-Receptors**

The in-vitro determination of the affinity of the inventive substituted pyrazoline compounds to $CB_1$/$CB_2$-Rezeptors is carried out as described in the publication of Ruth A. Ross, Heather C. Brockie et al., "Agonist-inverse agonist characterization at CB1 and CB2 cannabinoid receptors of L-759633, L759656 and AM630", British Journal of Pharmacology, 126, 665-672, (1999), whereby the transfected human $CB_1$ and $CB_2$ receptors of Receptor Biology, Inc. are used. The radioligand used for both receptors is [$^3$H]-CP55940. The respective parts of the description is hereby incorporated by reference and forms part of the present disclosure.

**II. In-vivo bioassay system for determination of cannabinoid activity**

**Mouse tetrad model**

Substances with affinity for cannabinoid receptors are known to produce a wide range of pharmacological effects. It is also known that intravenous administration of a substance with affinity for cannabinoid receptors in mice produces analgesia , hypothermia, sedation and catalepsy. Individually, none of these effects can be considered as proof that a tested substance has affinity for cannabinoid-receptors, since all of these effects are common for various classes of centrally active agents. However, substances, which show all of these effects, i.e. substances that are active in this so-called tetrad model are considered to have affinity for the cannabinoid receptors. It has further been shown that cannabinoid receptor antagonists are higly effective in blocking the effects of a cannabinoid agonist in the mouse tetrad model.

The tetrad model is described, for example, in the publication of A. C. Howlett et al, International Union of Pharmacology XXVII. Classification of Cannabinoid Receptors, Pharmacol Rev 54, 161-202 , 2002 and David R. Compton et al., "In-vivo Characterization of a Specific Cannabinoid Receptor Antagonist (SR141716A) :Inhibition of Tetrahydrocannbinol- induced Responses and Apparent Agonist Activity", J. Pharmacol. Exp. Ther. 277 , 2, 586-594, 1996. The corresponding parts of the description are hereby incorporated by reference.

**Material and Methods**

Male NMRI mice with a weight of 20-30 g (Harlan, Barcelona, Spain) are used in all of the following experiments.

Before testing in the behavioral procedures given below, mice are acclimatized to the experimental setting. Pre-Treatment control values are determined for analgesia hot plate latency (in seconds), rectal temperature, sedation and catalepsy.

In order to determine the agonistic activty of the substance to be tested, the mice are injected intravenously with the substance to be tested or the vehicle alone. 15 minutes after injection, latency in hot plate analgesia is measured. Rectal temperature, sedation and catalepsy are measured 20 minutes after injection.

In order to determine the antagonistic activity the identical procedure is used as for the determination of the agonistic effects, but with the difference that the substance to be evaluated for its antagonistic activity is injectected 5 minutes before the intravenous injection of 1.25 mg/kg Win-55,212 a known cannabinoid-receptor agonist.

### Hot plate analgesia

The hot plate analgesia is determined according to the method described in Woolfe D. et al. "The evaluation of analgesic action of pethidine hydrochloride (Demerol)", J. Pharmacol. Exp. Ther. 80, 300-307,1944. The respective description is hereby incorporated by reference and forms part of the present disclosure.

The mice are placed on a hot plate (Harvard Analgesimeter) at 55 $\pm$ 0.5 ˚C until they show a painful sensation by licking their paws or jumping and the time for these sensations to occur is recorded. This reading is considered the basal value (B). The maximum time limit the mice are allowed to remain on the hot plate in absence of any painful response is 40 seconds in order to prevent skin damage. This period is called the cut-off time (PC).

Fifteen minuts after the administration of the substance to be tested, the mice are again placed on the hot plate and the afore described procedure is repeated. This period is called the post-treatment reading (PT).

The degree of analgesia is calculated from the formula :

$$\%\ \text{MPE of Analgesia} = (\text{PT- B}) / (\text{PC-B}) \times 100$$

MPE = Maximum possible effect.

### Determination of sedation and ataxia

Sedation and ataxia is determined according to the method described in Desmet L. K. C. et al. "Anticonvulsive properties of Cinarizine and Flunarizine in Rats and Mice", Arzneim. -Forsch. (Frug Res) 25, 9, 1975. The respective description is hereby incorporated by reference and forms part of the present disclosure.

The chosen scoring system is

- **0:** no ataxia;
- **1:** doubful;
- **2:** obvious calmness and quiet;
- **3** pronounced ataxia;

prior to as well as after treatment.

The percentage of sedation is determined according to the formula:

$$\%\ \text{of sedation} = \text{arithmetic mean} / 3 \times 100$$

### Hypothermia:

Hypothermia is determined according to the method described in David R. Compton et al. "In-vivo Characterization of a Specific Cannabinoid Receptor Antagonist (SR141716A) Inhibition of Tetrahydrocannbinol- induced Responses and Apparent Agonist Activity", J. Pharmacol Exp Ther. 277 , 2, 586-594, 1996. The respective description is hereby incorporated by reference and forms part of the present disclosure.

The base-line rectal temperatures are determined with a thermometer (Yello Springs Instruments Co., Panlabs) and a thermistor probe inserted to 25mm before the administration of the substance to be tested. Rectal temperature

is again measured 20 minutes after the administration of the substances to be tested. The temperature difference is calculated for each animal, whereby differences of ≥-2 ˚C are considered to represent activity.

**Catalepsy:**

Catalepsy is determined according to the method described in Alpermann H. G. et al. "Pharmacological effets of Hoe 249: A new potential antidepressant", Drugs Dev. Res. 25, 267-282. 1992. The respective description is hereby incorporated by reference and forms part of the present disclosure.

The cataleptic effect of the substance to be tested is evaluated according to the duration of catalepsy, whereby the animals are placed head downwards with their kinlegs upon the top of the wooden block.

The chosen scoring system is:

Catalepsy for:

more than 60 seconds = 6; 50 -60 seconds = 5, 40-50 seconds = 4, 30-40 seconds = 3, 20-30 seconds = 2, 5-10 seconds = 1, and less than 5 seconds =0.

The percentage of catalepsy is determined according ot the following formula:

$$\% \text{ Catalepsy} = \text{arithmetic mean} / 6 \times 100$$

**III. In vivo testing for antiobesic activity**

The in-vivo testing for antiobesic activity of the inventive pyrazoline compounds is carried out as described in the publication of G. Colombo et al., "Appetite Suppression and Weight Loss after the Cannabinoid Antagonist SR 141716"; Life Sciences, 63 (8), 113-117, (1998). The respective part of the description is hereby incorporated by reference and forms part of the present disclosure.

**IV. In vivo testing for antidepressant activity**

The in-vivo testing for antidepressant activity of the inventive pyrazoline compounds in the water despair test is carried out as described in the publication of E.T. Tzavara et al., "The CB1 receptor antagonist SR141716A selectively increases monoaminergic neurotransmission in the medial prefrontal cortex: implications for therapeutic actions"; Br. J. Pharmacol. 2003, 138(4):544:53. The respective part of the description is hereby incorporated by reference and forms part of the present disclosure.

**V. Determination of the effect of continued administration of the inventive compounds on body weight in rats**

The weight of the animals was measured for 36 days, and the data grouped into 3 periods for analysis: Period 1, days 1-8 (period for adaptation of the animals to the environmental conditions: one week without treatment plus first day of treatment); Period 2, days 9-22 (treatment period: two weeks); Period 3, days 23-36 (escape treatment period: two weeks). The administration of the drugs started on day 8, immediately after the animal was weighted, and finished on day 21. The treatment period ranges between one day after first administration and one day after last administration. The drugs were administered i.p. once daily. For comparative purposes, absolute weights were transformed into relative percentages (each weight was divided by that of day 8 and multiplied by 100). Three treatment groups were constructed: vehicle, racemic N-piperidinyl-5-(4-chlorophenyl)-1-(2,4-dichlorophenyl)-4,5-dihydro-1H-pyrazole-3-carboxamide (10 mg/kg), and (R)-N-piperidinyl-5-(4-chlorophenyl)-1-(2,4-dichlorophenyl)-4,5-dihydro-1 H-pyrazole-3-carboxamide (10 mg/kg). The number of animals was 10 for the (R)-N-pipeddinyl-5-(4-chlorophenyl)-1-(2,4-dichlorophenyl)-4,5-dihydro-1H-pyrazole-3-carboxamide group and 9 for the racemic N-piperidinyl-5-(4-chlorophenyl)-1-(2,4-dichlorophenyl)-4,5-dihydro-1H-pyrazole-3-carboxamide group and for the vehicle group. In the vehicle group one animal died whereas in the racemic group one animal was excluded because its weight decreased during 5 days in the post-treatment period. All results are expressed as mean ± S.E.M.

The dependence of body-weight (expressed as a percentage relative to day 8) on time (number of days after inclusion in the study) and treatment was assessed by a two-way (time, treatment) analysis of variance (ANOVA) with

repeated measures on the time factor. The analysis was performed on periods 2 and 3, separately, and considering the first or second week in each period. The differences between pairs of curves (treatments) were analyzed using appropriate contrasts within the ANOVA test. The possible interaction between time and treatment was also considered including the Greenhouse-Geisser (G-G) correction. A value of $P < 0.05$ was considered significant.

**Figure 1** shows the effect of continued administration (i.p. once daily) of racemic N-piperidinyl-5-(4-chlorophenyl)-1-(2,4-dichlorophenyl)-4,5-dihydro-1 H-pyrazole-3-carboxamide **(A),** (R)-N-piperidinyl-5-(4-chlorophenyl)-1-(2,4-dichlorophenyl)-4,5-dihydro-1H-pyrazole-3-carboxamide **(B)** and vehicle on body weight in rats. The R-compound shows a stronger action than the racemate as well as a slower recovery of weight.

The present invention is illustrated below with the aid of examples. These illustrations are given solely by way of example and do not limit the general spirit of the present invention.

**Examples:**

**Abbreviations:**

| | |
|---|---|
| Eq. | Equivalent |
| Proc. | Process |
| Solv. Cryst. | Solvent used in crystallisation experiment |
| T Cryst. | Temperature at which crystallisation experiment was carried out |
| $1^{st}$ Cryst. | first crystallisation |
| $2^{nd}$ Crys. | second crystallisation |
| r.t. | room temperature (≈ 20-25 ˚C) |

**Example 1:**

**General synthesis of racemic N-piperidinyl-5-(4-chlorophenyl)-1-(2,4-dichlorophenyl)-4,5-dihydro-1H-pyrazole-3-carboxamide**

**a) 4-(4-chlorophenyl)-2-oxo-3-butenoic acid**

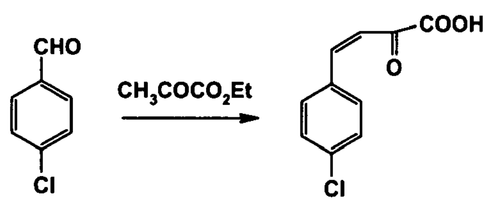


In a three neck flask p-chlorobenzaldehyde (13,3 g, 95 mmoles) and ethyl pyruvate (10 g, 86 mmoles) were dissolved in 150 ml of absolute ethanol. The solution was ice-cooled to 0˚C and an aqueous solution of NaOH (3.8 g in 45 mL water) was added dropwise keeping the temperature below or equal to 10˚C, whereby a yellow-orange colored precipitate was formed. The reaction mixture was stirred for 1 hour at 0˚C and an additional 1.5 hours at room temperature (approximately 25 ˚C). Afterwards the reaction mixture was cooled down to approximately 5˚C and the insoluble sodium salt of 4-(4-chlorophenyl)-2-oxo-3-butenoic acid was isolated by filtration.

The filtrate was left in the refrigerator overnight, whereby more precipitate is formed, which was filtered off, combined with the first fraction of the salt and washed with diethyl ether. The sodium salt of 4-(4-chlorophenyl)-2-oxo-3-butenoic acid was then treated with a solution of 2N HCl, stirred for some minutes and solid 4-(4-chlorophenyl)-2-oxo-3-butenoic acid was separated via filtration and dried to give 12.7 g of the desired product (70% of theoretical yield).

IR (KBr, $cm^{-1}$): 3500-2500, 1719,3, 1686,5, 1603,4, 1587,8, 1081,9. $^1$H NMR($CDCl_3$, δ) : 7,4 (d, J=8,4Hz, 2H), 7,5 (d, J=16,1Hz, 1 H), 7,6 (d, J=8,4Hz, 2H), 8,1(d, J=16,1Hz, 1H).

**b) 5-(4-chlorophenyl)-1-(2,4-dichlorophenyl)-4,5-dihydro-pyrazole-3-carboxylic acid**

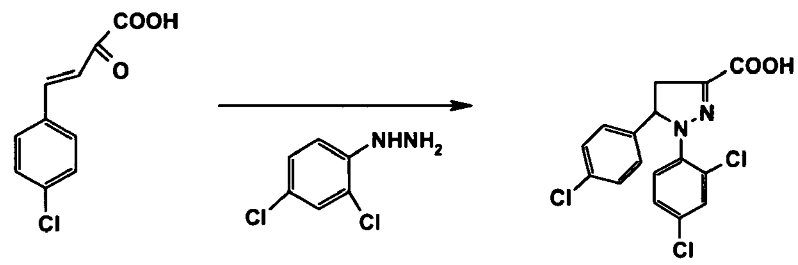

4-(4-chlorophenyl)-2-oxo-3-butenoic acid obtained according to step a) (12.6 g, 60 mmoles), 2,4-dichlorophenylhydrazine hydrochloride (12.8 g, 60 mmoles) and glacial acetic acid (200 mL) were mixed under a nitrogen atmosphere and heated to reflux for 4 hours, cooled down to room temperature (approximately 25 °C) and given into ice-water, whereby a sticky mass was obtained, which was extracted with methylene chloride. The combined methylene chloride fractions were washed with water, dried with sodium sulfate, filtered and evaporated to dryness to give a pale yellow solid (12.7 g, 57% of theoretical yield).

IR (KBr, $cm^{-1}$) : 3200-2200, 1668,4, 1458, 1251,4, 1104,8. $^1H$ NMR ($CDCl_3$, $\delta$) : 3,3 (dd, 1 H), 3,7 (dd, 1 H), 5,9 (dd, 1 H), 7,09-7,25 (m, 7H).

**(c) 5-(4-chlorophenyl)-1-(2,4-dichlorophenyl)-4,5-dihydro-pyrazole-3-carboxylic acid chloride**

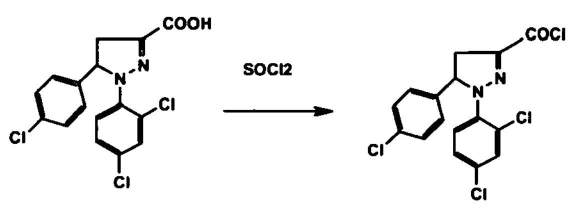


Under nitrogen atmosphere 5-(4-chlorophenyl)-1-(2,4-dichlorophenyl)-4,5-dihydro-pyrazole-3-carboxylic acid (2.5 g, 6.8 mmols) obtained according to step (b) was dissolved in 4 mL of in thionyl chloride and heated to reflux for 2.5 hours. The excess thionyl chloride is removed from the reaction mixture under reduced pressure and the resulting crude residue (2.6 g) is used without any further purification.

IR (KBr, $cm^{-1}$): 1732,3, 1700, 1533,3, 1478,1, 1212,9, 826,6.

**d) N-piperidinyl-5-(4-chlorophenyl)-1-(2,4-dichlorophenyl)-4,5-dihydropyrazole-3-carboxamide** [this compound may also be referred to as 5-(4-Chloro-phenyl)-1-(2,4-dichloro-phenyl)-4,5-dihydro-1 H-pyrazole-3-carboxylic acid piperidin-1-ylamide or as 1-(2,4-dichlorophenyl)-5-(4-chlorophenyl)-4,5-dihydro-N-(piperidin-1-yl)-1H-pyrazole-3-carboxamide]

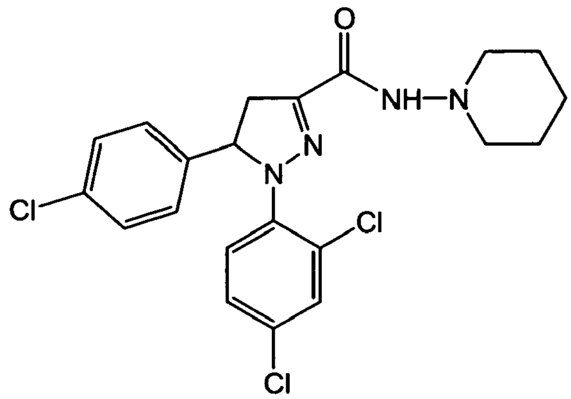

Under nitrogen atmosphere N-aminopiperidine (0.6 mL, 5.6 mmoles) and triethylamine (4 mL) were dissolved in methylene chloride (25 mL). The resulting mixture was ice-cooled down to 0˚C and a solution of 5-(4-chlorophenyl)-1-(2,4-dichlorophenyl)-4,5-dihydro-pyrazole-3-carboxylic acid chloride obtained in step (c) in methylene chloride (15 mL) was added dropwise. The resulting reaction mixture was stirred at room temperature (approximately 25 ˚C) overnight. Afterwards the reaction mixture was washed with water, followed by a saturated aqueous solution of sodium bicarbonate, then again with water, dried over sodium sulfate, filtered and evaporated to dryness in a rotavapor. The resulting crude solid was crystallized from ethanol. The crystallized solid was removed via filtration and the mother liquors were concentrated to yield a second fraction of crystallized product. The two fractions were combined to give a total amount of 1.7 g (57% of theoretical yield) of N-piperidinyl-5-(4-chlorophenyl)-1-(2,4-dichlorophenyl)-4,5-dihydropyrazole-3-carboxamide having a melting point of 183-186˚C.

IR (KBr, $cm^{-1}$) : 3222,9, 2934,9, 1647,4, 1474,7, 1268,3, 815,6.

$^1H$ NMR ($CDCl_3$, δ) : 1,4 (m, 2H), 1,7 (m, 4H), 2,8 (m, 4H), 3,3 (dd, J=6,1 y 18,3Hz, 1 H), 3,7 (dd, J=12,5 and 18,3 Hz, 1H). 5,7 (dd, J=6,1 and 12,5 Hz, 1 H), 7,0-7,2 (m, 6H), 7,4 (s, 1 H).

The resolution of the racemate of N-piperidinyl-5-(4-chlorophenyl)-1-(2,4-dichlorophenyl)-4,5-dihydro-1H-pyrazole-3-carboxamide may be carried out by methods known to those skilled in the art, e.g. quiral column chromatography.

However, is has been found by the present inventors that its intermediate, namely racemic 5-(4-chlorophenyl)-1-(2,4-dichlorophenyl)-4,5-dihydro-pyrazole-3-carboxylic acid may easily be separated into the respective enantiomers via reaction with a chiral base. The process for this resolution is described below.

**Example 2:**

**Resolution of the enantiomers of 5-(4-chlorophenyl)-1-(2,4-dichlorophenyl)-4,5-dihydro-pyrazole-3-carboxylic acid**

The resolution of the enantiomers of 5-(4-chlorophenyl)-1-(2,4-dichlorophenyl)-4,5-dihydro-pyrazole-3-carboxylic acid was carried out via reaction with the following chiral bases:

- Brucine
- Quinine
- (-)-Cinchonidine
- (+)-Cinchonine
- R-(+)-1-Phenylethylamine
- (1 R,2S)-(-)-Ephedrine hydrochloride
- (1S,2R)-(+)-Ephedrine hydrochloride.

In each case the reactions were carried out with 0.5 and 1 equivalents of base in respect to 1 equivalent of the acid compound and by using the following solvents

- Ethanol
- Acetone

- Acetonitril
- Dioxane
- Ethylacetate
- Chloroform.

The results are summarized in the following tables. It may be understood that the afore mentioned crystallisation experiments that are not reflected in the following tables did not yield crystals of the respective salts under the given conditions.

However, suitable conditions for crystallization of these salts can be determined by those skilled in the art via routine experiments.

In the following tables

Acid represents racemic 5-(4-chlorophenyl)-1-(2,4-dichlorophenyl)-4,5-dihydro-pyrazole-3-carboxylic acid

R-Acid represents the respective derivative of (R)-5-(4-chlorophenyl)-1-(2,4-dichlorophenyl)-4,5-dihydro-pyrazole-3-carboxylic acid

S-Acid represents the respective derivative of (S)-5-(4-chlorophenyl)-1-(2,4-dichlorophenyl)-4,5-dihydro-pyrazole-3-carboxylic acid

**Processes for crystallisation:**

**Process A:** A solution of the chiral base was added on top of a solution of the racemic acid at room temperature.

**Process C:** A solution of the racemic acid was added on top of a solution of the chiral base. The mixture was heated to reflux and solvent was added until dissolution was complete. The solution was left to crystallisation at r.t.

**Process D:** The chiral base was directly added on top of a solution of the racemic acid at room temperature.

**Process E:** The chiral base was directly added on top of a solution of the racemic acid at reflux temperature.

**Process F:** The solution of the salt was evaporated to dryness. The residue was dissolved in a minimum amount of the solvent under reflux heating. The solution was left to crystallisation at r.t.

**Resolution with Brucine**

Brucine

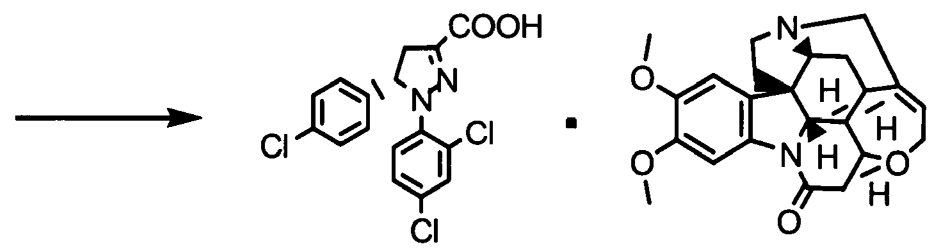

| Acid g (mmol) | Eq. amine | Proc. | Solvent for crystallisation | T Cryst. | Yield 1st Cryst. % | % S-Acid | % R-Acid | Yield 2nd Cryst. % | % S-Acid |
|---|---|---|---|---|---|---|---|---|---|
| 0,4 g (1,09 mmol) | 0,5 | A | 8 ml acetone | r.t. | 44,6 | 98,8 | 1,2 | 5,6% | 99,4 |
| 0,4 g (1,09 mmol) | 0,5 | A | 11,5 ml acetonitrile | r.t. | 50,7 | 47,5 | 52,4 | | |
| 0,4 g (1,09 mmol) | 1 | A | 17 ml acetonitrile | r.t. | 25,6 | 45,7 | 54,3 | | |

**Resolution with Quinine**

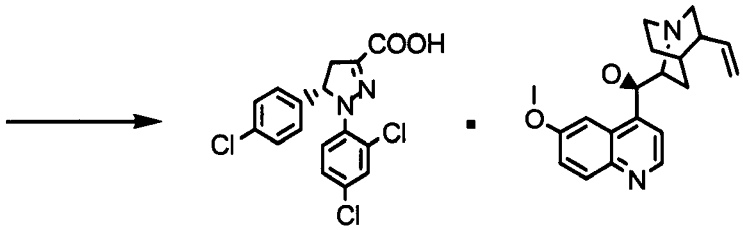

| Acid g (mmol) | Eq. amine | Proc. | Solvent for crystallisation | T Crys. | Yield 1st Cryst. % | % S-Acid | % R-Acid |
|---|---|---|---|---|---|---|---|
| 0,4 g (1,09 mmol) | 1 | A | 8 ml dioxane | r.t. | 49,46 | 91,6 | 8,3 |
| 0,4 g (1,09 mmol) | 1 | F | 15ml acetonitrile | r.t. | 26 | 94,4 | 5,5 |
| 0,4 g (1,09 mmol) | 1 | F | 2ml ethylacetate | r.t. | 25 | 96,7 | 3,3 |
| 0,4 g (1,09 mmol) | 0,5 | A | 4ml dioxane | r.t. | 38,5 | 97,5 | 2,5 |

**Resolution with (-)-Cinchonidine**

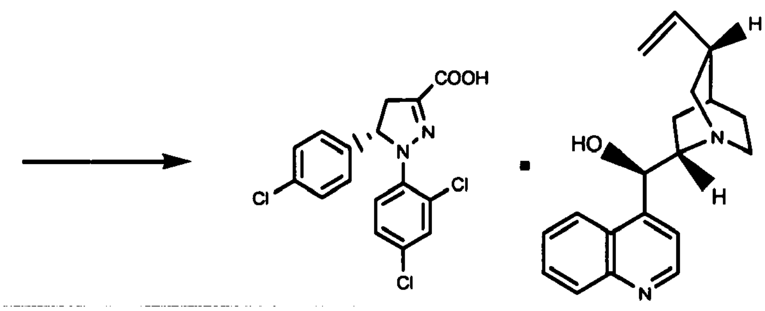

| Acid g (mmol) | Eq. amine | Proc. | Solvent for crystallisation | T Cryst. | Yield 1st Cryst. % | % S-Acid | % R-Acid |
|---|---|---|---|---|---|---|---|
| 0,4 g (1,09 mmol) | 1 | F | 2ml dioxane | r.t. | 31 | 94,4 | 5,6 |
| 0,4 g (1,09 mmol) | 1 | F | 19ml Ethylacetate | r.t. | 28,5 | 95,8 | 4,2 |

(continued)

| Acid g (mmol) | Eq. amine | Proc. | Solvent for crystallisation | T Cryst. | Yield 1st Cryst. % | % S-Acid | % R-Acid |
|---|---|---|---|---|---|---|---|
| 0,4 g (1,09 mmol) | 1 | F | 20ml acetone | r.t. | 19,6 | 96,9 | 3,1 |
| 0,4 g (1,09 mmol) | 1 | F | 24ml acetonitrile | r.t. | 42 | 85,8 | 14,1 |

**Resolution with (+)-Cinchonine**

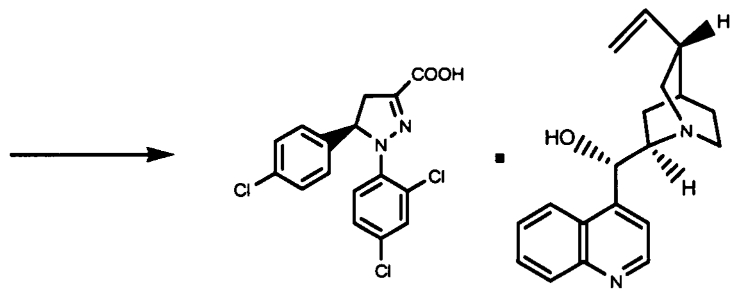

| Acid g (mmol) | Eq. amine | Proc. | Solvent for crystallisation | T Cryst . | Yield 1st Cryst. % | % S-Acid | % R-Acid |
|---|---|---|---|---|---|---|---|
| 0,4 (1,09 mmol) | 1 | C | 50 ml acetone | r.t. | 32,8 | 7,42 | 92,57 |
| 0,4 (1,09 mmol) | 0,5 | F | 30ml acetone | r.t. | 23,5 | 4,0 | 95,9 |
| 0,4 (1,09 mmol) | 0,5 | C | 50 ml acetonitrile | r.t. | 31,5 | 3,07 | 96,92 |
| 0,4 (1,09 mmol) | 1 | C | 50 ml acetonitrile | r.t. | 78,25 | 39,01 | 60,98 |
| 0,4 (1,09 mmol) | 0,5 | C | 15 ml ethylacetate | r.t. | 14,9 | 3,62 | 96,37 |
| 0,4 (1,09 mmol) | 1 | C | 27 ml ethylacetate | r.t. | 35 | 7,78 | 92,21 |
| 0,4 (1,09 mmol) | 1 | F | 4ml dioxane | r.t. | 21 | 33,5 | 66,4 |

**Resolution with R-(+)-1-Phenylethylamine**

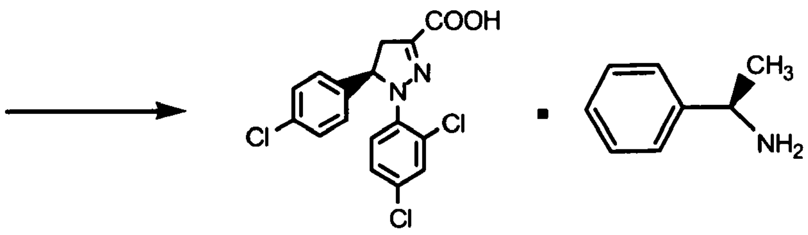

| Acid g (mmol) | Eq. amine | Proc. | Solvent for crystallisation | T Cryst. | Yield 1st Cryst. % | % S-Acid | % R-Acid |
|---|---|---|---|---|---|---|---|
| 0,4 (1,09 mmol) | 1 | D | 1,6ml etanol | r.t. | 22 | 51,4 | 48,6 |
| 0,4 (1,09 mmol) | 0,5 | E | 6ml acetonitrilo | r.t. | 11 | 8,1 | 91,9 |
| 0,4 (1,09 mmol) | 1 | E | 6ml acetonitrilo | r.t. | 50 | 36,8 | 63,2 |
| 0,4 (1,09 mmol) | 1 | E | 6ml dioxano | ≈5°C | 5 | 4,6 | 95,3 |

**Detailed description:**

**Resolution of 5-(4-chlorophenyl)-1-(2,4-dichlorophenyl)-4,5-dihydro-pyrazole-3-carboxylic acid with (+)-Cinchonine**

35 g (294.68 mmol) of racemic 5-(4-chlorophenyl)-1-(2,4-dichlorophenyl)-4,5-dihydro-pyrazole-3-carboxylic acid in 630 m acetonitril were added to a suspension of 16.39 g (47.34 mmol) of (+)-Cinchonine in 920 ml acetonitrile under vigorous stirring. The resulting suspension was heated to reflux and acetonitrile (1300 ml) was added until dissolution was complete. The resulting solution was left to crystalize at room temperature over the weekend, whereupon a crystalline solid was obtained. The crystalline solid was filtered off, washed with 50 ml of cold acetonitrile and dried to give 25.26 g of a white solid (ee ≈ 91-92%). No second fraction of crystals could be obtained from the mother liquors, neither upon cooling in the refrigerator nor upon concentration. Thus, recrystallisation of the diastereomeric salt was carried out in different solvents in order to improve the enantiomeric excess:

| Solvent | Yield | ratio of enantiomers % (R) / % (S) |
|---|---|---|
| Acetonitrile | 90 % | 99,6 / 0,4 |
| EtOH-$H_2O$ | 68,5 % | 99,3 / 0,7 |
| Isopropanol | 55 % | 98,9 / 1,1 |

Consequently, the product was recrystallized from acetonitrile to give 22.6 g of crystals (yield 72 mol-% related to chiral base). The ratio of enantiomers determined by capilar electrophoresis was:

99.7 % of (R)-5-(4-chlorophenyl)-1-(2,4-dichlorophenyl)-4,5-dihydro-pyrazole-3-carboxylic acid (+)-Cinchonine
0.3 % of (S)-5-(4-chlorophenyl)-1-(2,4-dichlorophenyl)-4,5-dihydro-pyrazole-3-carboxylic acid (+)-Cinchonine
ee = 99.4 %.

**Preparation of (R)-5-(4-chlorophenyl)-1-(2,4-dichlorophenyl)-4,5-dihydro-pyrazole-3-carboxylic acid from addition compound (R)-5-(4-chlorophenyl)-1-(2,4-dichlorophenyl)-4,5-dihydro-pyrazole-3-carboxylic acid-(+)-Cinchonine**

22.59 g (34 mmol) of the diastereomeric salt, which was recrystallized from acetonitrile, were suspended in 200 ml of 6N HCl and stirred at room temperature for 15 minutes. Afterwards, 200 ml of toluene were added until dissolution was complete. The mixture was stirred for 30 minutes and the phases were separated. The aqueous phase was extracted with toluene, the combined organic phases were washed with water, dried over sodium sulfate, filtered and evaporated to dryness, whereupon 11.9 g (95 %) of a white microcrystalline solid (melting point 129-133˚C) were obtained.

Enantiomeric excess determined by capilar electrophoresis. ee = 99.2 %
Chemical purity determined by HPLC: 99,3 %
$[\alpha]_D$ (c=1,23˚C, MeOH) = -429.

**Isolation of the enantiomer (R)-5-(4-chlorophenyl)-1-(2,4-dichlorophenyl)-4,5-dihydro-pyrazole-3-carboxylic acid from the mother liquors obtained from the crystallisation of the enantiomer (S)-5-(4-chlorophenyl)-1-(2,4-dichlorophenyl)-4,5-dihydro-pyrazole-3-carboxylic acid with Brucine**

The residue obtained from the evaporation of the mother liquors obtained from the crystallisation of (S)-5-(4-chlorophenyl)-1-(2,4-dichlorophenyl)-4,5-dihydro-pyrazole-3-carboxylic acid with Brucine was treated with 6N HCl as described above for obtaining the free acid. After evaporating the organic phase 21.86 g of a mixture with a theoretical composition of 20 mmoles (S)-5-(4-chlorophenyl)-1-(2,4-dichlorophenyl)-4,5-dihydro-pyrazole-3-carboxylic acid and 39.9 mmoles of (R)-5-(4-chlorophenyl)-1-(2,4-dichlorophenyl)-4,5-dihydro-pyrazole-3-carboxylic acid were obtained. A suspension of 13.82 g (39.9 mmoles) of (+)-cinchonine in 1000 ml of acetonitrile were added and the mixture was heated to reflux. More acetonitrile was added until dissolution was complete (total volume of acetonitrile 4000 ml). The mixture was then slowly cooled to room temperature to obtain crystals. After a few hours a white solid was obtained, which was filtered off and dried to give 22.42 (yield 84,5% related to the salt of (R)-5-(4-chlorophenyl)-1-(2,4-dichlorophenyl)-4,5-dihydro-pyrazole-3-carboxylic acid).

Enantiomeric excess as determined by capilar electrophoresis was ee = 92 %.

The purity of the isolated salt was comparable to the one of the salt obtained directly from the resolution of the racemate. Consequently, the same purification process as described above could be applied.

**Resolution of racemic 5-(4-chlorophenyl)-1-(2,4-dichlorophenyl)-4,5-dihydro-pyrazole-3-carboxylic acid with Brucine**

**Preparation of the diastereomeric salt with Brucine**

At room temperature 15.74 g (39.9 mmol) of Brucine were dissolved in 475 ml of acetone under vigorous stirring. A solution of 29.5 g (79.8 mmol) of the racemic 5-(4-chlorophenyl)-1-(2,4-dichlorophenyl)-4,5-dihydro-pyrazole-3-carboxylic acid in 85 ml acetone was added. The resulting mixture was allowed to crystallize for a couple of days, whereupon a crystalline solid was obtained. The crystalline solid was filtered off, washed with cold acetone and dried to yield 13.66 g (45 mol % related to the base) of a beige-coloured solid were obtained.

The ratio of the enantiomers as determined by capilar electrophoresis was:

> 99 % of (S)-5-(4-chlorophenyl)-1-(2,4-dichlorophenyl)-4,5-dihydro-pyrazole-3-carboxylic acid Brucine
> 1 % of (R)-5-(4-chlorophenyl)-1-(2,4-dichlorophenyl)-4,5-dihydro-pyrazole-3-carboxylic acid Brucine
> Enantiomeric excess ee = 98 %

The filtered mother liquors were left in the refrigerator and yielded a second fraction of crystals, which were filtered off and dried to give another 1.56 g (5.1 molar % related to the base).

The analysis of the 2nd fraction via capillar electrophoresis gave
99.3 % of (S)-5-(4-chlorophenyl)-1-(2,4-dichlorophenyl)-4,5-dihydro-pyrazole-3-carboxylic acid Brucine
0.7 % of (R)-5-(4-chlorophenyl)-1-(2,4-dichlorophenyl)-4,5-dihydro-pyrazole-3-carboxylic acid Brucine
Enantiomeric excess ee = 98.6 %

Total yield:
15.22 g (50.1 mol% related to base) of the sal with ee = 98 %.

**Determination of the chirality of the Brucine salt via X-ray crystallography**

The chirality of the enantiomer that crystallized with Brucine (ee 98 %) was determined via x-ray crystallography and corresponds to the S-enantiomer. Accordingly, the other enantiomer that crystallized with (+)-cinchonine is the corresponding R-enantiomer.

**Preparation of (S)-5-(4-chlorophenyl)-1-(2,4-dichlorophenyl)-4,5-dihydro-pyrazole-3-carboxylic acid from addition compound (S)-5-(4-chlorophenyl)-1-(2,4-dichlorophenyl)-4,5-dihydro-pyrazole-3-carboxylic acid brucine**

15.17 g (19.85 mmol) of (S)-5-(4-chlorophenyl)-1-(2,4-dichlorophenyl)-4,5-dihydro-pyrazole-3-carboxylic acid Brucine were suspended in 150 ml of 6N HCl and the suspension was stirred for 10 minutes, followed by the addition of 150 ml of toluene upon which complete dissolution was observed. The resulting mixture was stirred for an additional 30 minutes under control with thin layer chromatography(TLC) (silica gel, $Cl_2CH_2$:MeOH 95:5). Afterwards, the phases were separated, the aqueous phase was extracted with toluene and the combined toluene phases were washed with water three times, dried over sodium sulfate, filtered and evaporated to dryness. 7 g (95,5 %) of an oil were obtained, which solidified upon addition of a small amount of diethylether to yield a white amorphous solid that showed a crystalline transformation under melting at 130-133 ˚C.
TLC($Cl_2CH_2$:MeOH 95:5) : $R_f$ = 0.3
The analysis of the enantiomers via capillar electrophoresis gave

99.1 % (S)-5-(4-chlorophenyl)-1-(2,4-dichlorophenyl)-4,5-dihydro-pyrazole-3-carboxylic acid
0.9 % (R)-5-(4-chlorophenyl)-1-(2,4-dichlorophenyl)-4,5-dihydro-pyrazole-3-carboxylic acid
Enantiomeric excess ee = 98.2 %
$[\alpha]_D$ (c=1,23˚C, MeOH) = -480.5.
Chemical purity determined by HPLC: 99.1 %

$^1$H-NMR ($CDCl_3$) δ ppm: 3,2 (dd, J=6,4 y 18,2 Hz, 1H), 3,7 (dd, J=12,7 y 18,2 Hz, 1 H), 5,85 (dd, J=6,4 y 12,6 Hz, 1 H), 7,0-7,2 (m, 7H).

**Isolation of the enantiomer (S)-5-(4-chlorophenyl)-1-(2,4-dichlorophenyl)-4,5-dihydro-pyrazole-3-carboxylic acid from the mother liquors obtained from the crystallisation of the other enantiomer (R)-5-(4-chlorophenyl)-1-(2,4-dichlorophenyl)-4,5-dihydro-pyrazole-3-carboxylic acid with (+)-cinchonine**

The residue obtained from the evaporation of the mother liquors obtained from the crystallisation of (R)-5-(4-chlorophenyl)-1-(2,4-dichlorophenyl)-4,5-dihydro-pyrazole-3-carboxylic acid with (+)-Cinchonine was treated with 6N HCl as described above for obtaining the free acid. After evaporating the organic phase 23.63 g of a mixture with a theoretical composition of 13.34 mmoles of (R)-5-(4-chlorophenyl)-1-(2,4-dichlorophenyl)-4,5-dihydro-pyrazole-3-carboxylic acid and 47.34 mmoles of (S)-5-(4-chlorophenyl)-1-(2,4-dichlorophenyl)-4,5-dihydro-pyrazole-3-carboxylic acid were obtained. Said mixture, dissolved in 118 ml of acetone, was added on top of a solution of 18.7 g (47.4 mmoles) of brucine in 650 ml of acetone, stirred for several minutes and left to crystallize at room temperature overnight. A creme-coloured solid was obtained that was filtered off and dried to yield 28 g (77.5 % relative to the (S)-salt).
Enantiomeric excess as determined by capilar electrophoresis was ee = 98.2 %
The purity of the isolated salt was comparable to the one of the salt obtained directly from the resolution of the racemate. Consequently, the same purification process as described above could be applied.

**Preparation of (R)-N-piperidinyl-5-(4-chlorophenyl)-1-(2,4-dichlorophenyl)-4,5-dihydro-1H-pyrazole-3-carboxamide**

9.94 g (26.89 mmol) of (R)-5-(4-chlorophenyl)-1-(2,4-dichlorophenyl)-4,5-dihydro pyrazole-3-carboxylic acid were dissolved in 95 ml of toluene, followed by the addition of 2.35 ml (32.3 mmoles) of thionyl chloride and 4 drops of DMF.Under anhydrous nitrogen atmosphere the mixture was heated to a temperature of 75-80˚ C under vigorous stirring for two hours and then allowed to cool to room temperature. Said acid chloride solution was then added dropwise to a solution of 3.47 ml (31.2 mmol) of N-Aminopiperidine, 15 ml (107.6 mmol) of triethylamine and 38 ml of anhydrous toluene, which was cooled to 0-5 ˚C, thereby keeping the temperature below 10 ˚C during the addition. The reaction mixture was stirred at room temperature overnight, whereupon a suspension was obtained. Water and Toluene were added to said suspension until dissolution was complete and the phases were separated. The aqueous phase was extracted with toluene, the combined organic phases washed with an NaOH solution (10 %) and water, dried over sodium

sulfate, filtered off and evaporated to dryness to yield 13.41 g of light yellow oil, which failed to crystallize. The oil was purified via column chromatography over silica gel (eluent: petrol ether ethylacetate 90: 10 to 60: 40) to give 10.5 g (83.5 %) of (R)-N-piperidinyl-5-(4-chlorophenyl)-1-(2,4-dichlorophenyl)-4,5-dihydro-1H-pyrazole-3-carboxamide in form of an amorphous solid of light yellow color (melting point 75-78 °C).

$^1$H-NMR (DMSO-$d_6$) $\delta$ ppm : 9,25 (s, 1 H), 7,5 (d, J=8,8Hz, 1 H), 7,4 (d, J=2,3Hz, 1 H), 7,3-7,25 (2d, J=8,8 and 8,5Hz, 3H), 7,1 (d, J=8,5Hz, 2H), 5,8 (dd, J=5,7 and 11,8 Hz, 1 H), 3,65 (dd, J=11,8 and 18,1 Hz, 1 H), 3,0 (dd, J=5,7 and 18,1 Hz, 1 H), 2,75 (m, 4H), 1,5 (m, 4H), 1,2 (m, 2H).

Analysis of the enantiomer via chiral HPLC: ee = 100 %
Chemical purity determined by HPLC: 98,5%
$[\alpha]_D$ (c=1, 23°C, MeOH) = - 293,5

**Preparation of the hydrochloride salt of (R)-N-piperidinyl-5-(4-chlorophenyl)-1-(2,4-dichlorophenyl)-4,5-dihydro-1H-pyrazole-3-carboxamide**

0.5 g (1.1 mmol) of (R)-N-piperidinyl-5-(4-chlorophenyl)-1-(2,4-dichlorophenyl)-4,5-dihydro-1H-pyrazole-3-carboxamide were dissolved in 10 ml isopropanol, cooled with ice and a saturated HCl-ethanol solution was added. The solution changed its color and a white solid formed, which was filtered off, washed with isopropanol:diethylether (1:1) and dried to yield 0.38 g (71 %) of a white solid with a melting point of 160-165 °C. The purity determined by HPLC was 99.3 %. Determination of chlorine: 7.15 % (98.5 % of the theoretical value).

$^1$H-NMR (DMSO-$d_6$) $\delta$ ppm : 10,3 (bs, 1 H), 7,5 (m, 2H), 7,3 (2d, J= 2,5 y 8,5Hz, 3H), 7,15 (d, J=8,5Hz, 2H), 5,8 (dd, J=6,3 y 12,0Hz, 1H), 3,7 (dd+$H_2O$), 3,05 (m, 5H), 1,7 (m, 4H), 1,4 (m, 2H).

## Claims

1. Substituted pyrazoline compounds of general formula I,

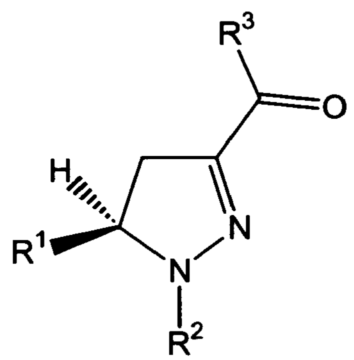

I

wherein

$R^1$ represents an optionally at least mono-substituted phenyl group;
$R^2$ represents an optionally at least mono-substituted phenyl group;
$R^3$ represents a saturated or unsaturated, optionally at least mono-substituted, optionally at least one heteroatom as ring member containing cycloaliphatic group, which may be condensed with an optionally at least mono-substituted mono- or polycyclic ring system; or $R^3$ represents an optionally at least mono-substituted aryl or heteroaryl group, which may be condensed with an optionally at least mono-substituted mono- or polycyclic ring system; or $R^3$ represents an $-NR^4R^5$-moiety,
$R^4$ and $R^5$, identical or different, represent a hydrogen atom; an unbranched or branched, saturated or unsaturated, optionally at least mono-substituted aliphatic radical; a saturated or unsaturated, optionally at least mono-substituted, optionally at least one heteroatom as ring member containing cycloaliphatic group, which may be condensed with an optionally at least mono-substituted mono- or polycyclic ring system; or an optionally at least mono-substituted aryl or heteroaryl group, which may be condensed with an optionally at least mono-substituted

mono- or polycyclic ring system and/or bonded via a linear or branched alkylene group; an $-SO_2-R^6$-moiety; or an - $NR^7R^8$-moiety,

$R^6$ represents a linear or branched, saturated or unsaturated, optionally at least mono-substituted aliphatic group; a saturated or unsaturated, optionally at least mono-substituted, optionally at least one heteroatom as ring member containing cycloaliphatic group, which may be condensed with a mono- or polycyclic ring-system; or an optionally at least mono-substituted aryl or heteroaryl group, which may be condensed with a mono- or polycyclic ring system and/or bonded via a linear or branched alkylene group;

$R^7$ and $R^8$, identical or different, represent a hydrogen atom; an unbranched or branched, saturated or unsaturated, optionally at least mono-substituted aliphatic radical; a saturated or unsaturated, optionally at least mono-substituted, optionally at least one heteroatom as ring member containing cycloaliphatic group, which may be condensed with an optionally at least mono-substituted mono- or polycyclic ring system; or an optionally at least mono-substituted aryl or heteroaryl group, which may be condensed with an optionally at least mono-substituted mono- or polycyclic ring system and/or bonded via a linear or branched alkylene group;

optionally in form of a corresponding N-oxide thereof, or a corresponding salt thereof, or a corresponding solvate thereof.

**2.** Compounds according to claim 1, **characterized in that** $R^1$ represents a phenyl group, which is optionally substituted by one or more substituents independently selected from the group consisting of a linear or branched $C_{1-6}$-alkyl group, a linear or branched $C_{1-6}$-alkoxy group, a halogen atom, $CH_2F$, $CHF_2$, $CF_3$, CN, OH, $NO_2$, -(C=O)-R', SH, SR', SOR', $SO_2R'$, $NH_2$, NHR', NR'R'', -(C=O)-$NH_2$, -(C=O)-NHR' and -(C=O)-NR'R'' whereby R' and R'' for each substituent independently represent linear or branched $C_{1-6}$ alkyl, preferably $R^1$ represents a phenyl group, which is optionally substituted by one or more substituents selected from the group consisting of methyl, ethyl, F, Cl, Br and $CF_3$, more preferably $R^1$ represents a phenyl group, which is mono-substituted with a chlorine atom in the 4-position.

**3.** Compounds according to claim 1 or 2, **characterized in that** $R^2$ represents a phenyl group, which is optionally substituted by one or more substituents independently selected from the group consisting of a linear or branched $C_{1-6}$-alkyl group, a linear or branched $C_{1-6}$-alkoxy group, a halogen atom, $CH_2F$, $CHF_2$, $CF_3$, CN, OH, $NO_2$, -(C=O)-R', SH, SR', SOR', $SO_2R'$, $NH_2$, NHR', NR'R'', -(C=O)-$NH_2$, -(C=O)-NHR' and -(C=O)-NR'R'', whereby R' and optionally R'' for each substituent independently represent linear or branched $C_{1-6}$ alkyl, preferably $R^2$ represents a phenyl group, which is optionally substituted by one or more substituents independently selected from the group consisting of methyl, ethyl, F, Cl, Br and $CF_3$, more preferably $R^2$ represents a phenyl group, which is di-substituted with two chlorine atoms in its 2- and 4-position.

**4.** Compounds according to one or more of claims 1-3, **characterized in that** $R^3$ represents a saturated or unsaturated, optionally at least mono-substituted, optionally at least one heteroatom as ring member containing $C_{3-8}$ cycloaliphatic group, which may be condensed with an optionally at least mono-substituted mono- or polycyclic ring system, or $R^3$ represents an optionally at least mono-substituted, 5- or 6-membered aryl or heteroaryl group, which may be condensed with an optionally at least mono-substituted mono- or polycyclic ring system, or $R^3$ represents an -$NR^4R^5$-moiety, preferably $R^3$ represents a saturated, optionally at least mono-substituted, optionally one or more nitrogen-atoms as ring member containing $C_{3-8}$ cycloaliphatic group, which may be condensed with an optionally at least mono-substituted mono- or polycyclic ring system, or $R^3$ represents an - $NR^4R^5$-moiety, more preferably $R^3$ represents a pyrrolidinyl group, a piperidinyl group or a piperazinyl group, whereby each of these groups may be substituted with one or more $C_{1-6}$-alkyl groups, or $R^3$ represents an -$NR^4R^5$-moiety.

**5.** Compounds according to one or more of claims 1-4, **characterized in that** $R^4$ and $R^5$, identical or different, represent a hydrogen atom; an unbranched or branched, saturated or unsaturated, optionally at least mono-substituted $C_{1-6}$-aliphatic radical; a saturated or unsaturated, optionally at least mono-substituted, optionally at least one heteroatom as ring member containing $C_{3-8}$-cycloaliphatic group, which may be condensed with an optionally at least mono-substituted mono- or polycyclic ring system; or an optionally at least mono-substituted, 5- or 6-membered aryl or heteroaryl group, which may be condensed with an optionally at least mono-substituted mono- or polycyclic ring system and/or bonded via a methylene ($-CH_2-$) or ethylene ($-CH_2-CH_2-$)-group; an —$SO_2-R^6$-moiety; or an -$NR^7R^8$-moiety, preferably one of these residues $R^4$ and $R^5$ represents a hydrogen atom and the other one of these residues $R^4$ and $R^5$ represents a saturated or unsaturated, optionally at least mono-substituted, optionally at least one heteroatom as ring member containing $C_{3-8}$-cycloaliphatic group, which may be condensed with an optionally at least mono-substituted mono- or polycyclic ring system; or an optionally at least mono-substituted, 5- or 6-membered aryl or heteroaryl group, which may be condensed with an optionally at least mono-substituted mono- or polycyclic ring system; an $-SO_2-R^6$-moiety; or an -$NR^7R^8$-moiety, or $R^4$ and $R^5$, identical or different, each represent

a $C_{1-6}$ alkyl group, more preferably one of these residues $R^4$ and $R^5$ represents a hydrogen atom and the other one of these residues $R^4$ and $R^5$ represents an optionally at least mono-substituted pyrrolidinyl group; an optionally at least mono-substituted piperidinyl group; an optionally at least mono-substituted piperazinyl group; an optionally at least mono-substituted triazolyl group; an —$SO_2$-$R^6$-moiety; or an - $NR^7R^8$-moiety, or $R^4$ and $R^5$, identical or different, represent a methyl group, an ethyl group, an n-propyl group, an isopropyl group, an n-butyl group, a sec-butyl group or a tert.-butyl group.

**6.** Compounds according to one or more of claims 1-5, **characterized in that** $R^6$ represents a linear or branched, saturated or unsaturated, optionally at least mono-substituted $C_{1-6}$ aliphatic group; a saturated or unsaturated, optionally at least mono-substituted, optionally at least one heteroatom as ring member containing $C_{3-8}$ cycloaliphatic group, which may be condensed with a mono- or polycyclic ring-system; or an optionally at least mono-substituted, 5- or 6-membered aryl or heteroaryl group, which may be condensed with a mono- or polycyclic ring system and/or bonded via a methylene (-$CH_2$-) or ethylene (-$CH_2$-$CH_2$)-group, preferably $R^6$ represents a $C_{1-6}$-alkyl group; a saturated, optionally at least mono-substituted cycloaliphatic group, which may be condensed with a mono- or polycyclic ring-system; or a phenyl group, which is optionally substituted with one or more $C_{1-6}$ alkyl groups.

**7.** Compounds according to one or more of claims 1-6, **characterized in that** $R^7$ and $R^8$, identical or different, represent a hydrogen atom; an unbranched or branched, saturated or unsaturated, optionally at least mono-substituted $C_{1-6}$ aliphatic radical; a saturated or unsaturated, optionally at least mono-substituted, optionally at least one heteroatom as ring member containing $C_{3-8}$ cycloaliphatic group, which may be condensed with an optionally at least mono-substituted mono- or polycyclic ring system; or an optionally at least mono-substituted, 5- or 6 membered aryl or heteroaryl group, which may be condensed with an optionally at least mono-substituted mono- or polycyclic ring system and/or bonded via a methylene (-$CH_2$-) or ethylene (-$CH_2$-$CH_2$)-group, preferably $R^7$ and $R^8$, identical or different, represent a hydrogen atom; or a $C_{1-6}$ alkyl radical.

**8.** Compounds according to one or more of claims 1-7 of general formula I

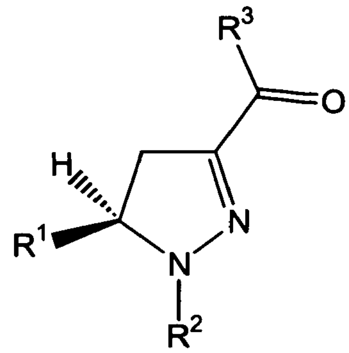

I

wherein

$R^1$ represents a phenyl group, which is optionally substituted by one or more substituents independently selected from the group consisting of methyl, ethyl, F, Cl, Br and $CF_3$,
$R^2$ represents a phenyl group, which is optionally substituted by one or more substituents independently selected from the group consisting of methyl, ethyl, F, Cl, Br and $CF_3$,
$R^3$ represents a pyrrolidinyl group, a piperidinyl group or a piperazinyl group, whereby each of these groups may be substituted with one or more $C_{1-6}$-alkyl groups, or $R^3$ represents an -$NR^4R^5$-moiety,
one of the residues $R^4$ and $R^5$ represents a hydrogen atom and the other one of these residues $R^4$ and $R^5$ represents an optionally at least mono-substituted pyrrolidinyl group; an optionally at least mono-substituted piperidinyl group; an optionally at least mono-substituted piperazinyl group; an optionally at least mono-substituted triazolyl group; an —$SO_2$-$R^6$-moiety; or an -$NR^7R^8$-moiety, or $R^4$ and $R^5$, identical or different, represent a methyl group, an ethyl group, an n-propyl group, an isopropyl group, an n-butyl group, a sec-butyl group or

a tert.-butyl group,
$R^6$ represents a $C_{1-6}$-alkyl group; a saturated, optionally at least mono-substituted cycloaliphatic group, which may be condensed with a mono- or polycyclic ring-system; or a phenyl group, which is optionally substituted with one or more $C_{1-6}$ alkyl groups, and
$R^7$ and $R^8$, identical or different, represent a hydrogen atom or a $C_{1-6}$ alkyl radical
optionally in form of a corresponding N-oxide thereof, or a corresponding salt thereof, or a corresponding solvate thereof.

**9.** Compounds according to one or more of claims 1-8 of general formula I

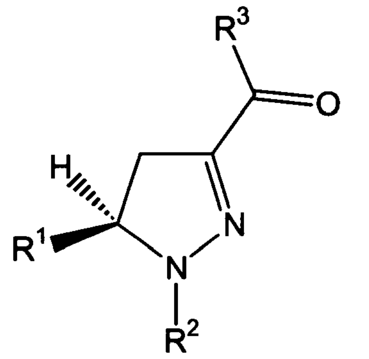

I

wherein

$R^1$ represents a phenyl group, which is optionally substituted with 1, 2, 3, 4 or 5 substituents independently selected from the group consisting of linear or branched $C_{1-6}$-alkyl, linear or branched $C_{1-6}$-alkoxy, F, Cl, Br, I, $CH_2F$, $CHF_2$, $CF_3$, CN, OH, $NO_2$, -(C=O)-R', SH, SR', SOR', $SO_2R'$ $NH_2$, NHR', NR'R", - (C=O)-$NH_2$, -(C=O)-NHR' and -(C=O)-NR'R", whereby R' and R" at each ocurrence independently represent a linear or branched $C_{1-6}$ alkyl group,
$R^2$ represents a phenyl group, which is optionally substituted with 1, 2, 3, 4 or 5 substituents independently selected from the group consisting of linear or branched $C_{1-6}$-alkyl, linear or branched $C_{1-6}$-alkoxy, F, Cl, Br, I, $CH_2F$, $CHF_2$, $CF_3$, CN, OH, $NO_2$, -(C=O)-R', SH, SR', SOR', $SO_2R'$, $NH_2$, NHR', NR'R", - (C=O)-$NH_2$, -(C=O)-NHR' and -(C=O)-NR'R", whereby R' and R" at each ocurrence independently represent a linear or branched $C_{1-6}$ alkyl group,
$R^3$ represents a saturated or unsaturated $C_{3-8}$ cycloaliphatic group, whereby said $C_{3-8}$ cycloaliphatic group is optionally substituted with 1, 2, 3 or 4 substituents independently selected from the group consisting of linear or branched $C_{1-6}$ alkyl, linear or branched $C_{1-6}$ alkoxy, OH, F, Cl, Br, I, CN, $CH_2F$, $CHF_2$, $CF_3$ and oxo (=O) and whereby said $C_{3-8}$ cycloaliphatic group may contain 1, 2 or 3 heteroatoms independently selected from the group consisting of N, O and S as ring members, or $R^3$ represents an -$NR^4R^5$-moiety,
$R^4$ represents a hydrogen atom or a linear or branched $C_{1-6}$-alkyl group,
$R^5$ represents a linear or branched $C_{1-6}$ alkyl group; an -$SO_2$-$R^6$-moiety; a saturated or unsaturated $C_{3-8}$ cycloaliphatic group, whereby said $C_{3-8}$ cycloaliphatic group is optionally substituted with 1, 2, 3 or 4 substituents independently selected from the group consisting of linear or branched $C_{1-6}$ alkyl group, a linear or branched $C_{1-6}$ alkoxy group, OH, F, Cl, Br, I, CN $CH_2F$, $CHF_2$, $CF_3$ and oxo (=O) and whereby said $C_{3-8}$ cycloaliphatic group may contain 1, 2 or 3 heteroatoms independently selected from the group consisting of N, O and S as ring members, and
$R^6$ represents a phenyl group, which is optionally substituted with 1, 2, 3, 4 or 5 substituents independently selected from the group consisting of a linear or branched $C_{1-6}$-alkyl group, a linear or branched $C_{1-6}$-alkoxy group, F, Cl, Br, I, $CH_2F$, $CHF_2$, $CF_3$, CN, OH, $NO_2$, -(C=O)-R', SH, SR', SOR', $SO_2R'$, $NH_2$, NHR', NR'R", -(C=O)-$NH_2$, -(C=O)-NHR' and —(C=O)-NR'R", whereby R' and R" at each ocurrence independently represent a linear or branched $C_{1-6}$ alkyl group,
optionally in form of a corresponding N-oxide thereof, or a corresponding salt thereof, or a corresponding solvate

thereof.

**10.** Compounds according to one or more of claims 1-9 of general formula I

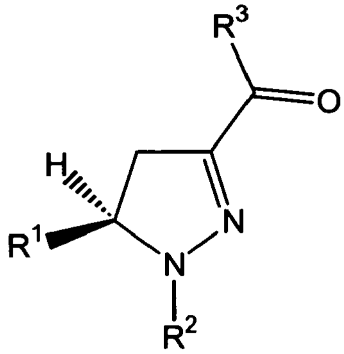

I

wherein

$R^1$ represents a phenyl group, which is optionally substituted with 1, 2, 3, 4 or 5 substituents independently selected from the group consisting of methyl, ethyl, F, Cl, Br and $CF_3$,
$R^2$ represents a phenyl group, which is optionally substituted with 1, 2, 3, 4 or 5 substituents independently selected from the group consisting of methyl, ethyl, F, Cl, Br and $CF_3$,
$R^3$ represents a pyrrolidinyl group, a piperidinyl group or a piperazinyl group, whereby each of these groups may be substituted with one or more of $C_{1-6}$-alkyl groups, or $R^3$ represents an $-NR^4R^5$-moiety,
$R^4$ represents a hydrogen atom or a linear or branched $C_{1-6}$-alkyl group,
$R^5$ represents a linear or branched $C_{1-6}$ alkyl group; an $-SO_2-R^6$-moiety; a pyrrolidinyl group; a piperidinyl group; a piperazinyl group; a homo-piperazinyl group; a morpholinyl group; a triazolyl group; whereby each of the heterocyclic rings may be substituted with one or more, identical or different, $C_{1-6}$-alkyl groups, and
$R^6$ represents a phenyl group, which is optionally substituted with one or more $C_{1-6}$ alkyl groups, which may be identical or different,
optionally in form of a corresponding N-oxide thereof, or a corresponding salt thereof, or a corresponding solvate thereof.

**11.** Compounds according to one or more of claims 1-10 of general formula I

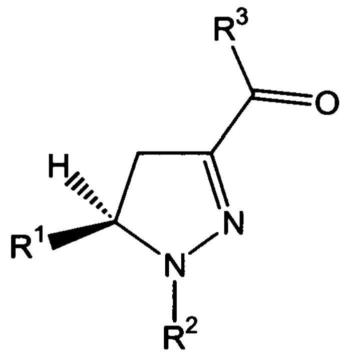

I

wherein

$R^1$ represents a phenyl ring, which is mono-substituted with a halogen atom, preferably a chlorine atom, in its 4-position,
$R^2$ represents a phenyl ring, which is di-substituted with two halogen atoms, preferably chlorine atoms, in its 2- and 4-position,
$R^3$ represents a pyrrolidinyl group, a piperidinyl group, a piperazinyl group, a homo-piperazinyl group, a morpholinyl group, or an —$NR^4R^5$-moiety,
$R^4$ represents a hydrogen atom or a linear or branched $C_{1-6}$-alkyl group,
$R^5$ represents a linear or branched $C_{1-6}$ alkyl group; an -$SO_2$-$R^6$-moiety; a pyrrolidinyl group; a piperidinyl group; a piperazinyl group; a homo-piperazinyl group; a morpholinyl group; or a triazolyl group whereby each of the heterocyclic rings may be substituted with one or more, identical or different, $C_{1-6}$-alkyl groups, and
$R^6$ represents a phenyl group, which is optionally substituted with one or more $C_{1-6}$ alkyl groups, which may be identical or different,
optionally in form of a corresponding N-oxide thereof, or a corresponding salt thereof, or a corresponding solvate thereof.

**12.** The compound (R)-N-piperidinyl-5-(4-chloro-phenyl)-1-(2,4-dichlorophenyl)-4,5-dihydro-1H-pyrazol-3-carboxamide according to one or more of claims 1 to 11:

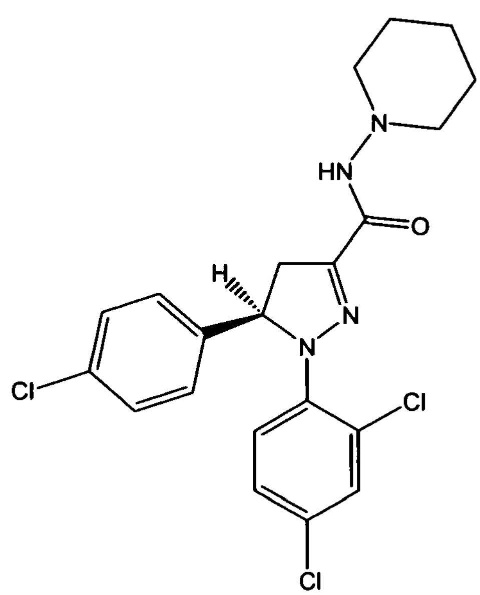

optionally in the form of a corresponding N-oxide, a corresponding salt or a corresponding solvate.

**13.** Process for the manufacture of substituted pyrazoline compounds of general formula I according to one or more of claims 1 to 12, **characterized in that** at least one compound of general formula IIa

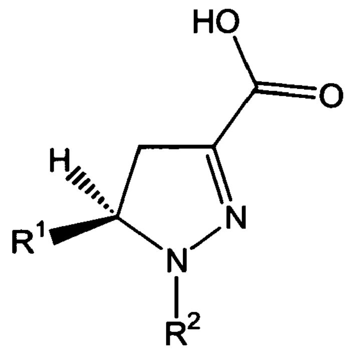

(IIa)

wherein $R^1$ and $R^2$ have the meaning according to one or more of claims 1-12, is optionally transferred under inert atmosphere to a compound of general formula (III) via reaction with an activating agent

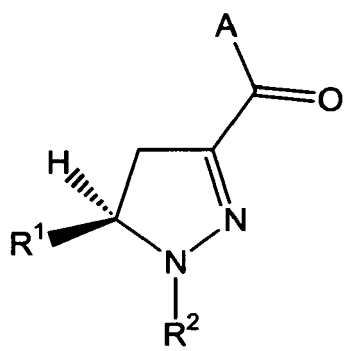

(III)

wherein the substituents $R^1$ and $R^2$ have the meaning given above and A represents a leaving group, preferably a chlorine atom, said compound being optionally isolated and/or optionally purified, and at least one compound of general formula (IIa) is reacted with a compound of general formula $R^3H$, wherein $R^3$ represents an $-NR^4R^5$-moiety, with $R^4$ and $R^5$ having the meaning according to one or more of claims 1-12, under inert atmosphere to yield a substituted pyrazoline compound of general formula I, wherein $R^3$ represents an $-NR^4R^5$-moiety,
or at least one compound of general formula (III) is reacted with a compound of the general formula $R^3H$, in which $R^3$ has the meaning according to one or more of claims 1-12 under inert atmosphere to yield a compound of general formula (I) according to one or more of claims 1-12, which is optionally isolated and/or optionally purified.

**14.** Process according to claim 13, wherein the compound IIa is obtained from a mixture comprising the enantiomers

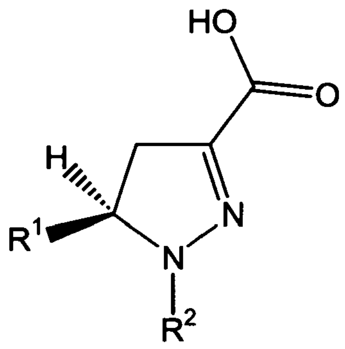

and

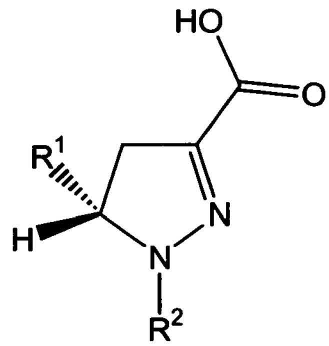

(IIa) (IIb).

**15.** Process according to claim 14, wherein the compound IIa is obtained from the mixture in form of an addition compound with a chiral base, preferably (+)-Cinchonine or R-(+)-1-Phenylethylamine, and preferably liberated from the addition compound.

**16.** Process according to claim 14 or 15, wherein the mixture comprising the enantiomers IIa and IIb is obtained by reacting at least one benzaldehyde compound of general formula IV

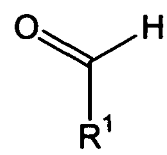

(IV)

wherein $R^1$ has the meaning according to one or more of claims 1-12, is reacted with a pyruvate compound of general formula (V)

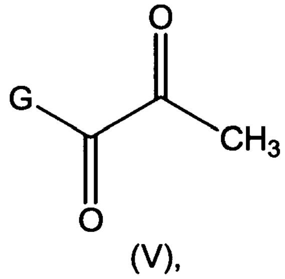

(V),

wherein G represents an OR group with R being a branched or unbranched $C_{1-6}$ alkyl radical or G represents an $O^-K$ group with K being a cation,
to yield a compound of general formula (VI)

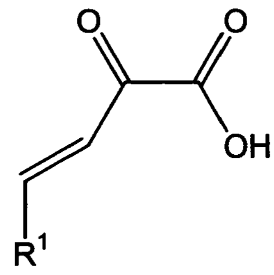

(VI)

wherein $R^1$ has the meaning given above, which is optionally isolated and/or optionally purified, and which is reacted with an optionally substituted phenyl hydrazine of general formula (VII)

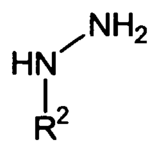

(VII)

or a corresponding salt thereof, wherein $R^2$ has the meaning according to one or more of claims 1-12, under inert atmosphere, to yield a mixture of compounds

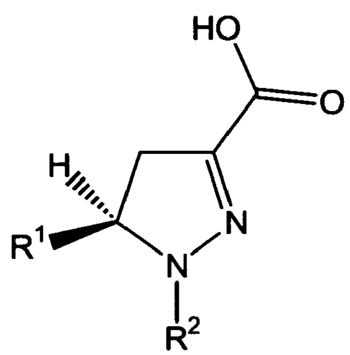

and

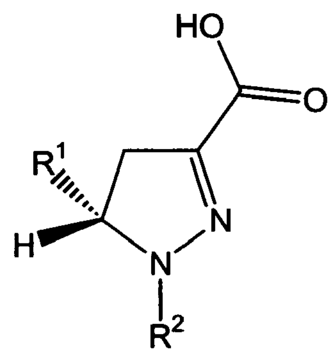

(IIa) (IIb).

**17.** Medicament comprising one or more substituted pyrazoline compounds of general formula I according to one or more of claims 1-12 and optionally one or more pharmaceutically acceptable excipients.

**18.** Medicament according to claim 17 for the modulation of cannabinoid-receptors, preferably cannabinoid 1 ($CB_1$) receptors, for the prophylaxis and/or treatment of disorders of the central nervous system, disorders of the immune

system, disorders of the cardiovascular system, disorders of the endocrinous system, disorders of the respiratory system, disorders of the gastrointestinal tract or reproductive disorders.

**19.** Medicament according to claim 17 or 18 for the prophylaxis and/or treatment of food intake disorders, preferably bulimia, anorexia, cachexia, obesity, type II diabetus mellitus (non-insuline dependent diabetes mellitus), more preferably obesity.

**20.** Medicament according to claim 17 or 18 for the prophylaxis and/or treatment of psychosis.

**21.** Medicament according claim 17 or 18 for the prophylaxis and/or treatment of alcohol abuse and/or alcohol addiction, nicotine abuse and/or nicotine addiction, drug abuse and/or drug addiction and/or medicament abuse and/or medicament addiction, preferably drug abuse and/or drug addiction and/or nicotine abuse and/or nicotine addiction.

**22.** Medicament according to claim 17 or 18 for the prophylaxis and/or treatment of cancer, preferably for the prophylaxis and/or treatment of one or more types of cancer selected from the group consisting of brain cancer, bone cancer, lip cancer, mouth cancer, esophageal cancer, stomach cancer, liver cancer, bladder cancer, pancreas cancer, ovary cancer, cervical cancer, lung cancer, breast cancer, skin cancer, colon cancer, bowel cancer and prostate cancer, more preferably for the prophylaxis and/or treatment of one or more types of cancer selected from the group consisting of colon cancer, bowel cancer and prostate cancer.

**23.** Medicament according to claim 17 or 18 for the prophylaxis and/or treatment of one or more disorders selected from the group consisting of bone disorders, preferably osteoporosis (e.g. osteoporosis associated with a genetic predisposition, sex hormone deficiency, or ageing), cancer-associated bone disease or Paget's disease of bone; schizophrenia, anxiety, depression, epilepsy, neurodegenerative disorders, cerebellar disorders, spinocerebellar disorders, cognitive disorders, cranial trauma, head trauma, stroke panic attacks, peripheric neuropathy, glaucoma, migraine, Morbus Parkinson, Morbus Huntington, Morbus Alzheimer, Raynaud's disease, tremblement disorders, compulsive disorders, senile dementia, thymic disorders, tardive dyskinesia, bipolar disorders, medicament-induced movement disorders, dystonia, endotoxemic shock, hemorragic shock, hypotension, insomnia, immunologic disorders, sclerotic plaques, vomiting, diarrhea, asthma, memory disorders, pruritus, pain, or for potentiation of the analgesic effect of narcotic and non-narcotic analgesics, or for influencing intestinal transit.

**24.** Use of at least one substituted pyrazoline compound according to one or more of claims 1-12 and optionally one or more pharmaceutically acceptable excipients, for the preparation of a medicament for the modulation of cannabinoid-receptors, preferably cannabinoid 1 ($CB_1$) receptors, for the prophylaxis and/or treatment of disorders of the central nervous system, disorders of the immune system, disorders of the cardiovascular system, disorders of the endocrinous system, disorders of the respiratory system, disorders of the gastrointestinal tract or reproductive disorders.

**25.** Use of at least one substituted pyrazoline compound according to one or more of claims 1-12 and optionally one or more pharmaceutically acceptable excipients, for the preparation of a medicament for the prophylaxis and/or treatment of food intake disorders, preferably bulimia, anorexia, cachexia, obesity, type II diabetus mellitus (non-insuline dependent diabetes mellitus), more preferably obesity.

**26.** Use of at least one substituted pyrazoline compound according to one or more of claims 1-12 and optionally one or more pharmaceutically acceptable excipients, for the preparation of a medicament for the prophylaxis and/or treatment of psychosis.

**27.** Use of at least one substituted pyrazoline compound according to one or more of claims 1-12 and optionally one or more pharmaceutically acceptable excipients, for the preparation of a medicament for the prophylaxis and/or treatment of alcohol abuse and/or alcohol addiction, nicotine abuse and/or nicotine addiction, drug abuse and/or drug addiction and/or medicament abuse and/or medicament addiction, preferably drug abuse and/or drug addiction and/or nicotine abuse and/or nicotine addiction.

**28.** Use of at least one substituted pyrazoline compound according to one or more of claims 1-12 and optionally one or more pharmaceutically acceptable excipients, for the preparation of a medicament for the prophylaxis and/or treatment of cancer, preferably for the prophylaxis and/or treatment of one or more types of cancer selected from the group consisting of brain cancer, bone cancer, lip cancer, mouth cancer, esophageal cancer, stomach cancer, liver cancer, bladder cancer, pancreas cancer, ovary cancer, cervical cancer, lung cancer, breast cancer, skin

cancer, colon cancer, bowel cancer and prostate cancer, more preferably for the prophylaxis and/or treatment of one or more types of cancer selected from the group consisting of colon cancer, bowel cancer and prostate cancer.

**29.** Use of at least one substituted pyrazoline compound according to one or more of claims 1-12 and optionally one or more pharmaceutically acceptable excipients, for the preparation of a medicament for the prophylaxis and/or treatment of one or more disorders selected from the group consisting of bone disorders, preferably osteoporosis (e.g. osteoporosis associated with a genetic predisposition, sex hormone deficiency, or ageing), cancer-associated bone disease or Paget's disease of bone; schizophrenia, anxiety, depression, epilepsy, neurodegenerative disorders, cerebellar disorders, spinocerebellar disorders, cognitive disorders, cranial trauma, head trauma, stroke, panic attacks, peripheric neuropathy, glaucoma, migraine, Morbus Parkinson, Morbus Huntington, Morbus Alzheimer, Raynaud's disease, tremblement disorders, compulsive disorders, senile dementia, thymic disorders, tardive dyskinesia, bipolar disorders, medicament-induced movement disorders, dystonia, endotoxemic shock, hemorragic shock, hypotension, insomnia, immunologic disorders, sclerotic plaques, vomiting, diarrhea, asthma, memory disorders, pruritus, pain, or for potentiation of the analgesic effect of narcotic and non-narcotic analgesics, or for influencing intestinal transit.

**30.** A method of modulating cannabinoid-receptors, preferably cannabinoid 1 ($CB_1$) receptors, of preventing and/or treating disorders of the central nervous system, disorders of the immune system, disorders of the cardiovascular system, disorders of the endocrinous system, disorders of the respiratory system, disorders of the gastrointestinal tract or reproductive disorders, comprising administering to a subject, preferably a human, a therapeutically effective amount of at least one substituted pyrazoline compound according to one or more of claims 1-12.

**31.** A method of treating food intake disorders, preferably bulimia, anorexia, cachexia, obesity, type II diabetus mellitus (non-insuline dependent diabetes mellitus), more preferably obesity, comprising administering to a subject, preferably a human, a therapeutically effective amount of at least one substituted pyrazoline compound according to one or more of claims 1-12.

**32.** A method of treating psychosis comprising administering to a subject, preferably a human, a therapeutically effective amount of at least one substituted pyrazoline compound according to one or more of claims 1-12.

**33.** A method of treating alcohol abuse and/or alcohol addiction, nicotine abuse and/or nicotine addiction, drug abuse and/or drug addiction and/or medicament abuse and/or medicament addiction, preferably drug abuse and/or drug addiction and/or nicotine abuse and/or nicotine addiction, comprising administering to a subject, preferably a human, a therapeutically effective amount of at least one substituted pyrazoline compound according to one or more of claims 1-12.

**34.** A method of treating of cancer, preferably for the prophylaxis and/or treatment of one or more types of cancer selected from the group consisting of brain cancer, bone cancer, lip cancer, mouth cancer, esophageal cancer, stomach cancer, liver cancer, bladder cancer, pancreas cancer, ovary cancer, cervical cancer, lung cancer, breast cancer, skin cancer, colon cancer, bowel cancer and prostate cancer, more preferably for the prophylaxis and/or treatment of one or more types of cancer selected from the group consisting of colon cancer, bowel cancer and prostate cancer comprising administering to a subject, preferably a human, a therapeutically effective amount of at least one substituted pyrazoline compound according to one or more of claims 1-12.

**35.** A method of treating one or more disorders selected from the group consisting of bone disorders, preferably osteoporosis (e.g. osteoporosis associated with a genetic predisposition, sex hormone deficiency, or ageing), cancer-associated bone disease or Paget's disease of bone; schizophrenia, anxiety, depression, epilepsy, neurodegenerative disorders, cerebellar disorders, spinocerebellar disorders, cognitive disorders, cranial trauma, head trauma, stroke panic attacks, peripheric neuropathy, glaucoma, migraine, Morbus Parkinson, Morbus Huntington, Morbus Alzheimer, Raynaud's disease, tremblement disorders, compulsive disorders, senile dementia, thymic disorders, tardive dyskinesia, bipolar disorders, medicament-induced movement disorders, dystonia, endotoxemic shock, hemorragic shock, hypotension, insomnia, immunologic disorders, sclerotic plaques, vomiting, diarrhea, asthma, memory disorders, pruritus, pain, or for potentiation of the analgesic effect of narcotic and non-narcotic analgesics, or for influencing intestinal transit, comprising administering to a subject, preferably a human, a therapeutically effective amount of at least one substituted pyrazoline compound according to one or more of claims 1-12.

Figure 1:

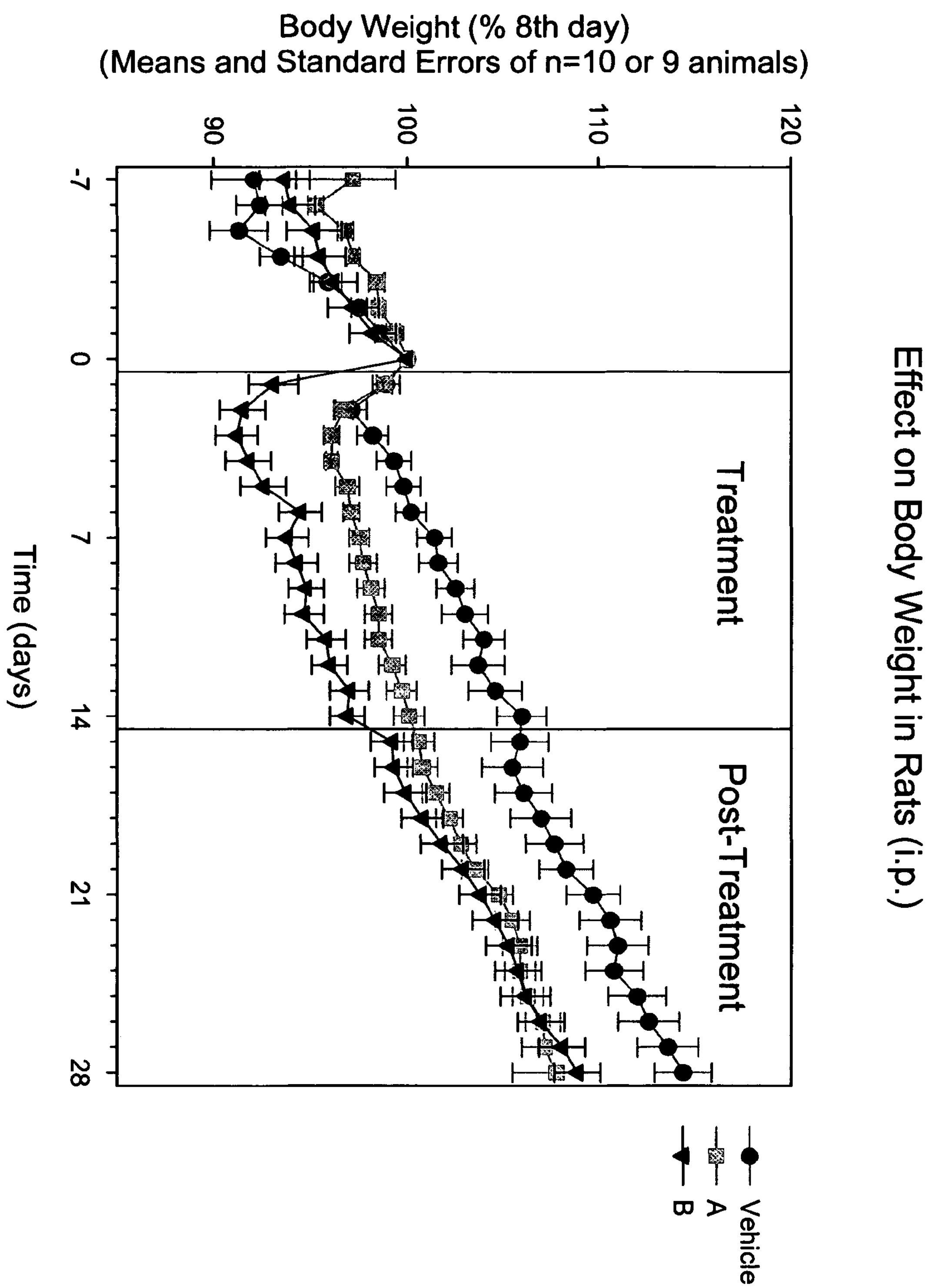
Effect on Body Weight in Rats (i.p.)
Body Weight (% 8th day)
(Means and Standard Errors of n=10 or 9 animals)
90
100
110
120
Treatment
Post-Treatment
-7
0
7
14
21
28
Time (days)
Vehicle
A
B

European Patent Office

# EUROPEAN SEARCH REPORT

Application Number
EP 05 38 4005

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 1 083 171 A (LABORATORIOS DEL DR. ESTEVE, S.A) 14 March 2001 (2001-03-14) * page 4, lines 17-22; examples 40-42; table 1 * | 1 | C07D231/06<br>A61K31/4155<br>A61P25/00 |
| Y | US 2005/096373 A1 (ALCON-MARRUGAT MONTSERRAT ET AL) 5 May 2005 (2005-05-05) * the whole document * | 1 | |
| Y | WO 02/076949 A (SOLVAY PHARMACEUTICALS B.V; LANGE, JOSEPHUS, H.M; KRUSE, CORNELIS, G;) 3 October 2002 (2002-10-03) * the whole document * | 1-35 | |
| Y | SHIM J-Y ET AL: "Molecular interaction of the antagonist N-(piperidin-1-yl)-5-(4-chlorophenyl)-1-(2,4-dichlorophenyl)-4-methyl-1H-pyrazole-3-carboxamide with the CB1 cannabinoid receptor" JOURNAL OF MEDICINAL CHEMISTRY, AMERICAN CHEMICAL SOCIETY. WASHINGTON, US, vol. 45, no. 7, March 2002 (2002-03), pages 1447-1459, XP002968557 ISSN: 0022-2623 * the whole document * | 1-35 | TECHNICAL FIELDS SEARCHED (IPC)<br>C07D<br>A61K<br>A61P |
| E | WO 2005/077911 A (LABORATORIOS DEL DR. ESTEVE S.A; CUBERES ALTISEN, ROSA; FRIGOLA CONSTA) 25 August 2005 (2005-08-25) * the whole document * | 1-35 | |

The present search report has been drawn up for all claims

| Place of search | Date of completion of the search | Examiner |
|---|---|---|
| Munich | 9 December 2005 | Lauro, P |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

2

EPO FORM 1503 03.82 (P04C01)

**ANNEX TO THE EUROPEAN SEARCH REPORT ON EUROPEAN PATENT APPLICATION NO.** EP 05 38 4005

This annex lists the patent family members relating to the patent documents cited in the above-mentioned European search report.
The members are as contained in the European Patent Office EDP file on
The European Patent Office is in no way liable for these particulars which are merely given for the purpose of information.

09-12-2005

| Patent document cited in search report | Publication date | Patent family member(s) | Publication date |
|---|---|---|---|
| EP 1083171 A | 14-03-2001 | AT 265437 T | 15-05-2004 |
| | | AU 752001 B2 | 05-09-2002 |
| | | AU 3932999 A | 20-12-1999 |
| | | BG 105005 A | 31-08-2001 |
| | | BR 9910801 A | 27-11-2001 |
| | | CA 2333475 A1 | 09-12-1999 |
| | | CN 1307566 A | 08-08-2001 |
| | | DE 69916828 D1 | 03-06-2004 |
| | | DE 69916828 T2 | 14-04-2005 |
| | | DK 1083171 T3 | 09-08-2004 |
| | | ES 2137138 A1 | 01-12-1999 |
| | | WO 9962884 A1 | 09-12-1999 |
| | | ES 2221382 T3 | 16-12-2004 |
| | | HU 0102102 A2 | 28-03-2002 |
| | | JP 2002516908 T | 11-06-2002 |
| | | LT 2000108 A | 25-09-2001 |
| | | LV 12632 A | 20-03-2001 |
| | | LV 12632 B | 20-07-2001 |
| | | NO 20006029 A | 26-01-2001 |
| | | NZ 508990 A | 20-12-2002 |
| | | PL 344412 A1 | 05-11-2001 |
| | | PT 1083171 T | 30-09-2004 |
| | | RU 2233272 C2 | 27-07-2004 |
| | | SI 20580 A | 31-12-2001 |
| | | SK 18072000 A3 | 10-07-2001 |
| | | TW 572898 B | 21-01-2004 |
| | | UA 61137 C2 | 15-03-2001 |
| | | US 6353117 B1 | 05-03-2002 |
| | | ZA 200007638 A | 13-11-2001 |
| US 2005096373 A1 | 05-05-2005 | US 2005096474 A1 | 05-05-2005 |
| WO 02076949 A | 03-10-2002 | AT 284872 T | 15-01-2005 |
| | | BR 0205602 A | 08-07-2003 |
| | | CA 2422708 A1 | 03-10-2002 |
| | | CN 1486301 A | 31-03-2004 |
| | | CZ 20030698 A3 | 18-06-2003 |
| | | DE 60202270 D1 | 20-01-2005 |
| | | DE 60202270 T2 | 19-05-2005 |
| | | ES 2229132 T3 | 16-04-2005 |
| | | HU 0303148 A2 | 28-01-2004 |
| | | JP 2004518763 T | 24-06-2004 |
| | | NO 20032892 A | 18-11-2003 |
| | | NZ 524633 A | 25-02-2005 |
| | | PL 363751 A1 | 29-11-2004 |
| | | PT 1373216 T | 31-05-2005 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 05 38 4005

This annex lists the patent family members relating to the patent documents cited in the above-mentioned European search report.
The members are as contained in the European Patent Office EDP file on
The European Patent Office is in no way liable for these particulars which are merely given for the purpose of information.

09-12-2005

| Patent document cited in search report | Publication date | Patent family member(s) | Publication date |
|---|---|---|---|
| WO 02076949 A | | SK 3082003 A3 | 07-10-2003 |
| | | ZA 200307322 A | 20-12-2004 |
| WO 2005077911 A | 25-08-2005 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **HOLLISTER.** *Pharm. Rev.,* 1986, vol. 38, 1-20
- **RENY ; SINGHA.** *Prog. Drug. Res.,* 1991, vol. 36, 71-114
- **CONSROE ; SANDYK.** Marijuana/Cannabinoids, Neurobiology and Neurophysiology. CRC Press, 1992, vol. 459
- *Synthetic communications,* 1996, vol. 26 (11), 2229-33
- **PASCUAL, A.** *J. Prakt Chem.,* 1999, vol. 341 (7), 695-700
- **LIN, S. et al.** *Heterocycles,* 2001, vol. 55 (2), 265-277
- **RAO, P. et al.** *J. Org. Chem.,* 2000, vol. 65 (22), 7323-7344
- **PEARSON D.E ; BUEHLER, C.A.** *Synthesis,* 1972, 533-542
- **KROGSGAARD-LARSEN et al.** Textbook of Drugdesign and Discovery. Taylor & Francis, April 2002
- **ECKEL et al.** *The Lancet,* 2005, vol. 365, 1415-1428
- **ALBERTI et al.** *Diabet. Med.,* 1998, vol. 15, 539-53
- *JAMA,* 2001, vol. 285, 2486-97
- **PAGOTTO ; PASQUALI.** *The Lancet,* 2005, vol. 365, 1363, 1364
- **RUTH A. ROSS ; HEATHER C. BROCKIE et al.** Agonist-inverse agonist characterization at CB1 and CB2 cannabinoid receptors of L-759633, L759656 and AM630. *British Journal of Pharmacology,* 1999, vol. 126, 665-672
- **A. C. HOWLETT et al.** International Union of Pharmacology XXVII. *Classification of Cannabinoid Receptors, Pharmacol Rev,* 2002, vol. 54, 161-202
- **DAVID R. COMPTON et al.** In-vivo Characterization of a Specific Cannabinoid Receptor Antagonist (SR141716A) :Inhibition of Tetrahydrocannbinol- induced Responses and Apparent Agonist Activity. *J. Pharmacol. Exp. Ther.,* 1996, vol. 277 (2), 586-594
- **WOOLFE D. et al.** The evaluation of analgesic action of pethidine hydrochloride (Demerol. *J. Pharmacol. Exp. Ther.,* 1944, vol. 80, 300-307
- **DESMET L. K. C. et al.** Anticonvulsive properties of Cinarizine and Flunarizine in Rats and Mice. *Arzneim. -Forsch. (Frug Res,* 25 September 1975
- **DAVID R. COMPTON et al.** In-vivo Characterization of a Specific Cannabinoid Receptor Antagonist (SR141716A) Inhibition of Tetrahydrocannbinol- induced Responses and Apparent Agonist Activity. *J. Pharmacol Exp Ther.,* 1996, vol. 277 (2), 586-594
- **ALPERMANN H. G. et al.** Pharmacological effets of Hoe 249: A new potential antidepressant. *Drugs Dev. Res.,* 1992, vol. 25, 267-282
- **G. COLOMBO et al.** Appetite Suppression and Weight Loss after the Cannabinoid Antagonist SR 141716. *Life Sciences,* 1998, vol. 63 (8), 113-117